# FASCICULE DE BREVET EUROPEEN

(11) **EP 0 863 887 B1**
(45) Date de publication et mention de la délivrance du brevet: **23.01.2002**
(21) Numéro de dépôt: 96934977.8
(22) Date de dépôt: 25.10.1996
(51) Int. Cl.: C07D 305/14, C07D 263/26, C07D 413/12, C07D 263/20, C07D 263/16, C07F 7/08, C07C 233/87, C07C 229/34

(54) **INTERMEDIAIRES POUR L'HEMISYNTHESE DE TAXANES ET LEURS PROCEDES DE PREPARATION**
ZWISCHERPRODUKTE FÜR DIE HEMISYNTHESE VON TAXANEN UND DEREN VERFAHREN ZU HERSTELLUNG
INTERMEDIARY COMPOUNDS FOR THE HEMISYNTHESIS OF TAXANES AND PREPARATION PROCESSES THEREFOR

(30) Priorité: 27.10.1995 FR 9512739
(43) Date de publication de la demande: 16.09.1998
(73) Titulaire: Societé d'Etude et de Recherche en Ingenierie Pharmaceutique Seripharm, 72000 Le Mans (FR)
(72) Inventeur: CHANTELOUP, Luc, F-72190 Sargé-lès-Le Mans (FR); CHAUVEAU, Bruno, 94, rue de Sargé F-72000 Le Mans (FR); CORBIN, Christine, F-72700 Etival-lès-Le Mans (FR); DHAL, Robert, Pruille-le-Chétif F-72700 Le Mans (FR); LE GUEN, Sonia, F-72000 Le Mans (FR); LAMY, Arnaud, F-72650 La Milesse (FR); LEZE, Antoine, F-72000 Le Mans (FR); ROBIN, Jean-Pierre, F-72000 Le Mans (FR)
(74) Mandataire: Le Pennec, Magali
(86) Numéro de dépôt international: FR9601676
(87) Numéro de publication internationale: WO9715562

(56) Documents cités:
- EP-A- 0 414 610
- EP-A- 0 522 958
- WO-A-91/13066
- WO-A-93/10076
- US-A- 4 876 399

## Description

La présente invention concerne de nouveaux intermédiaires pour l'hémisynthèse de taxanes et leurs procédés de préparation.

Les taxanes, substances naturelles dont le squelette diterpènique est généralement estérifié par une chaine latérale β-aminoacide dérivée de la N-alcoyl- ou N-aroyl-phényl-isosérine, sont connus comme agents anticancéreux. On compte plusieurs dizaines de taxanes isolés à partir de Taxacées du genre *Taxus*, comme par exemple, le PACLITAXEL (R₁ = Ac, R₂ = Ph, R₃ = R₄ = H), la céphalomanine, leurs dérivés désacétylés en position 10, ou les baccatines (dérivés sans chaîne latérale) représentés par les formules 1 et 2 ci-dessous.

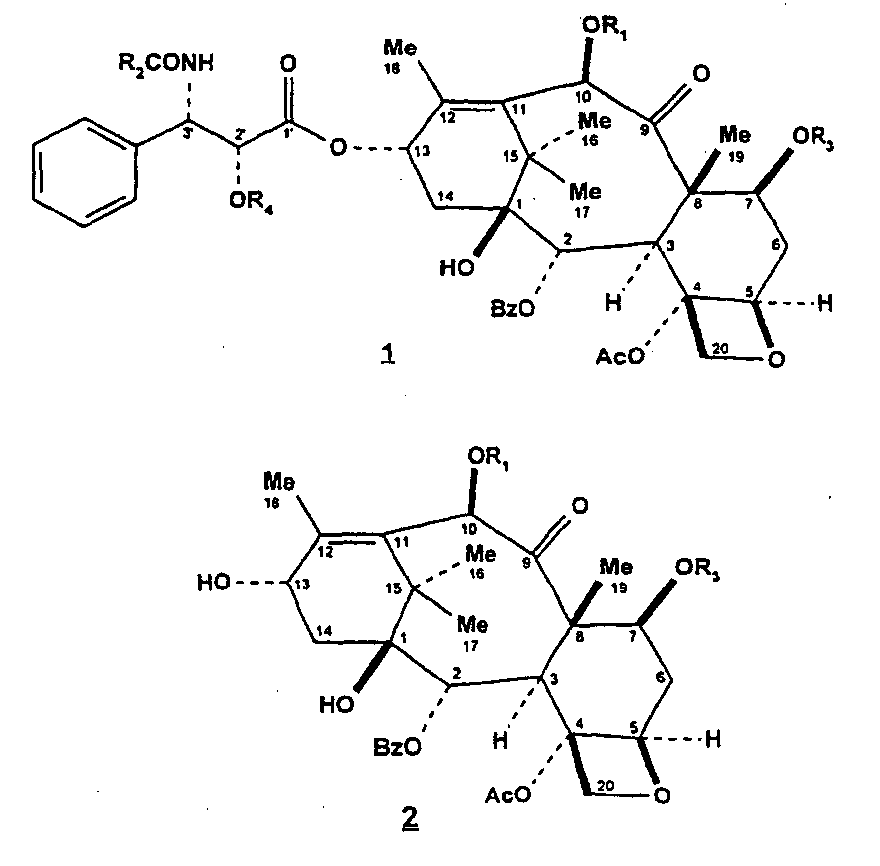

Dans le souci de ne pas épuiser rapidement sa source d'origine, *Taxus brevifolia*, des chercheurs français ont cherché à isoler le PACLITAXEL à partir de parties renouvelables (les feuilles) de *T*. *baccata*, l'if européen. Ils ont ainsi mis en évidence le précurseur biogénétique probable des taxanes, la 10-désacétylbaccatine III, tremplin de choix pour l'hémisynthèse, en raison de sa relative abondance dans les extraits de feuilles.

L'hémisynthèse des taxanes, comme le PACLITAXEL ou le DOCETAXEL (R₁ = Ac, R₂ = t.butyloxy, R₃= R₄ = H), consiste donc à estérifier l'hydroxy en 13 d'un dérivé protégé de la baccatine ou de la 10-désacétylbaccatine III, avec un dérivé d'acide β-aminé.

Différents procédés d'hémisynthèse du PACLITAXEL ou du DOCETAXEL sont décrits dans l'état de la technique (EP-0 253 738, EP-0 336 840, EP-0 336 841, EP-0 495 718, WO 92/09589, WO 94/07877, WO 94/07878, WO 94/07879, WO 94/10169, WO 94/12482, EP-0 400 971, EP-0 428 376, WO 94/14787). Deux ouvrages récents [I. Georg, T.T. Chen, I Ojima, and D.M. Vyas, "Taxane Anticancer Agents, Basic Science and Current Status", ACS Symposium Series 583, Washington (1995)] et surtout [Matthew Suffness, "TAXOL® Science and Applications" CRC press (1995) et 1500 références citées] comprennent des compilations exhaustives des hémisynthèses de taxanes.

Les chaînes latérales β-aminoacide dérivées de la N-alcoyl- ou N-aroylphénylisosérine du PACLITAXEL ou du DOCETAXEL sont de configuration (2R,3S), et l'une des principales difficultés de l'hémisynthèse des taxanes réside dans l'obtention d'un produit énantiomériquement pur. Le premier problème consiste à obtenir un énantiomère pur des dérivés de la phényl-isosérine employés dans l'hémisynthèse des taxanes. Le second problème consiste à conserver cette pureté énantiomérique au cours de l'estérification du dérivé de la baccatine et des traitements ultérieurs des produits obtenus (déprotection des hydroxyles, etc.).

De nombreux travaux de synthèse asymétriques mettant en jeu des dérivés d'acides β-aminés se sont focalisés sur la chimie de l'isosérine et de ses dérivés, β-aminoacides dont une forme cyclique déshydratée est une β-lactame (EP-O-525 589). La plupart des différentes synthèses de dérivés de la phényl-isosérine, utiles comme précurseurs de chaînes latérales de taxanes, convergent par un intermédiaire commun, l'acide cis-β-phényl-glycidique (2R,3R), qui est ensuite converti en β-phényl-isosérine par réaction avec l'ammoniac (EP-0 495 718) ou un nucléophile (Gou & coll., *J*. *Org. Chem.,* 1983, 58, 1287-89). Ces différents procédés nécessitent un nombre d'étapes nucléophile (Gou & coll., J. Org. Chem, 1983, 58, 1287-89). Ces différents procédés nécessitent un nombre d'étapes important pour obtenir la β-phényl-isosérine de configuration (2R,3S), avec nécessairement une étape de dédoublement de racémique par des techniques usuelles de cristallisation sélective, soit pour l'acide cis-β-phénylglycidique, soit pour la β-phényl-isosérine, ou ultérieurement, après transformation. Par ailleurs, pour préserver la pureté énantiomérique des précurseurs de chaînes latérales de taxanes au cours de l'estérification du dérivé de la baccatine, différents moyens ont été proposés, en particulier en utilisant des intermédiaires cycliques de configuration bloquée, qui écartent les risques d'isomérisation lors de réactions d'estérification dans des conditions réactionnelles dures. Il s'agit en particulier de dérivés de β-lactames (EP-0 400 971), d'oxazolidines (WO 92/09589, WO 94/07877, WO 94/07878, WO 94/07879, WO 94/10169, WO 94/12482), d'oxazinones (EP-0 428 376), ou encore d'oxazolines (WO 94/14787). Ces précurseurs cycliques sont préparés à partir du dérivé correspondant de la β-phényl-isosérine. Comme pour cette dernière, les procédés proposés impliquent un grand nombre d'étapes, et une nécessaire résolution de racémique pour obtenir le précurseur de chaîne latérale de taxanes recherché. Il était donc important de mettre au point une nouvelle voie de synthèse améliorée des intermédiaires précurseurs de chaîne latérale de taxanes, en particulier des énantiomères de l'acide cis-β-phényl-glycidique, de la β-phényl-isosérine et de leurs dérivés cycliques.

On peut citer parmi ces procédés :
La demande EP-0 522 958 décrit la préparation de taxoïdes par condensation d'un dérivé de la β-phénylisosérine sur un noyau baccatine ou 10-déacétylbaccatine protégé. Elle ne concerne pas la réaction de Ritter.
La demande de brevet WO 93/10076 qui décrit la préparation de Taxol par couplage d'une chaîne énantiomériquement pure avec un noyau taxane sans utiliser la réaction de Ritter.
La demande de brevet EP-0 414 610 décrit un procédé de préparation de la β-phénylisosérine énantiomériquement pure par action d'un azoture sur un oxirane. Le brevet ne décrit pas la condensation de la chaîne latérale sur le noyau taxane par une réaction de Ritter.
La demande WO 91/13066 décrit la préparation de l'acide cis-β-phényl-glycidique utilisé ultérieurement pour préparer la β-phénylisosérine. Le brevet ne décrit pas la condensation de la chaîne latérale sur le noyau taxane par la réaction de Ritter.

Enfin, pour l'hémisynthèse des taxanes, et en particulier du PACLITAXEL, le seul dérivé de la baccatine approprié utilisé jusqu'à présent est celui pour lequel le radical 7-hydroxy est protégé par un trialkyl-silyle (EP-0 336 840, WO 94/14787), dont la déprotection s'effectue exclusivement en milieu acide. Il était donc également important d'employer de nouveaux groupements protecteurs de la fonction hydroxy, permettant en particulier la protection sélective du radical 7-hydroxy, autorisant en outre un choix plus large de conditions opératoires pour l'étape de déprotection.

La présente invention concerne tout d'abord un procédé amélioré de préparation de précurseurs de chaîne latérale de taxanes.

Le procédé selon l'invention consiste à transformer un dérivé cis-β-aryl-glycidate de formule générale I
dans laquelle
Ar représente un aryle, en particulier le phényle et
R représente un radical hydrocarboné, de préférence un alkyle linéaire ou ramifié ou un cycloalkyle éventuellement substitué par un ou plusieurs groupes alkyles,
de manière à mettre en place régio- et stéréo-spécifiquement la β-N-alcoylamide et l'α-hydroxyle ou leurs précurseurs cycliques en une seule étape par une réaction de Ritter. Selon le milieu réactionnel, on distingue ainsi deux types de réactions de Ritter : l'une avec ouverture de l'oxétane conduisant à une forme linéaire de la chaîne directement et complètement fonctionnalisée, l'autre conduisant à la formation directe d'une oxazoline. Le symbole "*" indique la présence d'un carbone asymétrique, de configuration R ou S. Dans les deux cas, la réaction de Ritter est stéréospécifique, avec rétention de configuration en C-2, et inversion de configuration en C-3. D'une manière avantageuse, on effectue le procédé selon l'invention sur l'un des énantiomères du dérivé cis-β-aryl-glycidate de formule générale 1, de manière à obtenir l'énantiomère correspondant de la chaîne linéaire ou de l'oxazoline obtenue, sans nécessiter par la suite une résolution de racémique. Selon le mode de préparation du dérivé cis-β-aryl-glycidate de formule générale 1, décrit ultérieurement, R représente un énantiomère optiquement pur d'un radical hydrocarboné chiral de fort encombrement stérique, avantageusement un cycloalkyle substitué par un ou plusieurs groupes alkyle, en particulier un cyclohexyle, R sera alors de préférence l'un des énantiomères du radical menthyle, en particulier le (+)-menthyle.

### 1. Synthèse directe de la chaîne linéaire

La synthèse directe de la chaîne linéaire par la réaction de Ritter, consiste à faire réagir un dérivé cis-β-aryl-glycidate de formule générale I défini ci-dessus, avec un nitrile de formule

R₂-CN

dans laquelle
R₂ représente un radical aryle, de préférence un phényle, en présence d'un acide protonique tel les acides sulfuriques, perchloriques, tétrafluoroboriques, etc. et d'eau.

On obtient alors un dérivé de la β-aryl-isosérine de formule générale IIa, 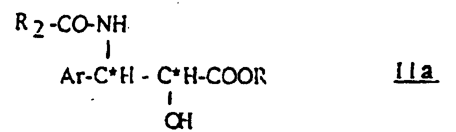 dans laquelle Ar, R et R₂ sont définis précédemment.

La réaction s'effectue avec une inversion de la configuration du C-3 du dérivé cis-β-phényl-glycidate. Ainsi, en partant d'un dérivé cis-β-phényl-glycidate (2R, 3R), on obtient le dérivé correspondant de la β-aryl-isosérine de configuration (2R, 3S).

On effectue la réaction de Ritter dans un solvant approprié, à une température comprise entre- 75 et + 25°C.

Le solvant approprié peut être le nitrile lui-même lorsqu'il est liquide à la température réactionnelle, ou bien l'acide lui-même (ac. sulfurique, perchlorique ou tétrafluoroborique), ou un solvant tel par exemple le chlorure de méthylène ou l'éther éthylique. Les acides protoniques classiquement utilisés peuvent contenir l'eau nécessaire à l'hydrolyse.

Lorsque l'on emploie le benzonitrile (R₂ = phényle) sur le cis-β-aryl-glycidate de formule générale I de configuration (2R, 3R) pour lequel Ar représente un phényle, on obtient alors directement le dérivé correspondant de la β-aryl-isosérine de formule générale IIa pour laquelle Ar et R2 représentent un phényle de configuration (2R, 3S), qui n'est autre que le précurseur de la chaine latérale du PACLITAXEL.

### 2. Synthèse directe de la chaîne cyclique

Pour cette seconde éventualité, on effectue également une réaction de Ritter, avec un nitrile de formule

R'₂-CN

dans laquelle
R'₂ représente R₂ défini précédemment, ou un radical alkyle inférieur ou perhalogénoalkyle inférieur, tel que le trichlorométhyle,
en présence d'un acide de Lewis, en particulier le complexe trifluorure de bore acide acétique, l'éthérate de trifluorure de bore, le pentachlorure d'antimoine, le tétrachlorure d'étain, le tétrachlorure de titane, etc., ou d'un acide protonique tel que par exemple l'acide tétrafluoroborique, la réaction étant conduite en milieu anhydre.

Comme pour la synthèse de la chaîne linéaire, le solvant peut être le nitrile lui-même lorsqu'il est liquide à la température réactionnelle, ou bien un solvant approprié tcl que par exemple le chlorure de méthylène ou l'éther éthylique. La température réactionnelle est également comprise entre - 75 et + 25°C.

En l'absence d'eau, on effectue une réaction de Ritter intramoléculaire, et l'on obtient l'oxazoline de formule générale IIb 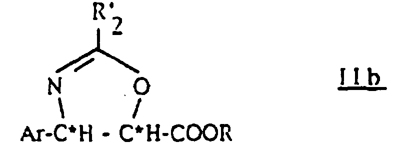 dans laquelle Ar, R et R'₂ sont définis précédemment.

Comme dans la réaction de Ritter en présence d'eau, la réaction s'effectue avec une inversion de la configuration du C-3 du dérivé cis-β-phényl-glycidate. Ainsi, en partant d'un dérivé cis-β-phényl-glycidate (2R,3R), on obtient l'oxazoline correspondante de configuration (2R,3S).

Pour les deux réactions de Ritter, afin d'éviter la formation d'un carbocation libre, cause de nombreuses réactions secondaires potentielles, on effectue de préférence l'addition des réactifs dans l'ordre ci-après : i) on forme d'abord le complexe entre le nitrile et l'acide, puis ii) le catalyseur acide est ajouté sur le mélange constitué de l'oxirane et du nitrile.

Les produits obtenus par cette première étapc, dérivés de la β-aryl-isosérine de formule générale IIa ou oxazoline de formule générale IIb, peuvent être de nouveau transformés dans une deuxième étape facultative décrite ci-après, ou alors convertis en acide par saponification ménagée, avant d'effectuer leur couplage sur un dérivé protégé de la baccatine pour l'hémisynthèse de taxanes, en particulier du PACLITAXEL et ses dérivés désacétylés en 10 ou du DOCETAXEL. Dans le cas de dérivés de la β-aryl-isosérine de formule générale IIa, on peut précéder la saponification d'une étape usuelle de protection de l'hydroxy par un groupe protecteur approprié. On obtient alors un dérivé de formule générale II'a 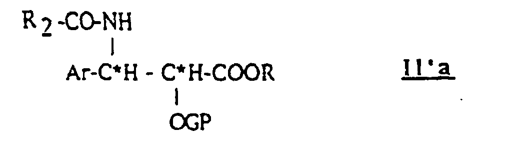 dans laquelle
Ar, R et R₂ sont définis précédemment, et
GP représente un groupe protecteur de la fonction hydroxy, approprié pour la synthèse des taxanes, en particulier choisi parmi les radicaux alkoxyéther, aralkoxyéther, aryloxyéther ou haloalkoxycarbonyle, tels que par exemple les groupes méthoxyméthyle, 1-éthoxyéthyle, benzyloxyméthyle, (β-triméthylsilyl-éthoxy)-méthyle, les radicaux tétrahydropyranyle, β-alkoxycarbonyle (TrOC), les éthers β-halogénés, alkylsilylés, ou les radicaux alkoxyacétyle, aryloxyacétyle, haloacétyle ou formyle.

### 3. Transformation éventuelle des dérivés de formule IIa ou IIb

Les dérivés de formule générale IIa ou llb obtenus précédemment, peuvent être éventuellement transformés en de nouveaux intermédiaires précurseurs de chaîne latérale dans l'hémisynthèse des taxanes. Ces transformations se font avec rétention de la configuration des C-2 et C-3. Les nouveaux intermédiaires obtenus auront donc la même stéréochimie que les dérivés de formule IIa ou IIb desquels ils sont issus. Les produits obtenus dans cette deuxième étape sont ensuite convertis en acide par saponification ménagée, avant d'effectuer leur couplage sur un dérivé protégé de la baccatine pour l'hémisynthèse de taxane, en particulier du l'ACLITAXEL ou du DOCETAXEL.

### 3.1 Cyclisation des dérivés de formule générale IIa

Les dérivés de formule générale IIa peuvent être ensuite transformés en oxazoline de formule IIb, selon les méthodes usuelles de l'état de la technique (WO 94/14787).

Les dérivés de la β-aryl-isosérine de formule générale IIa peuvent également être transformés en de nouveaux intermédiaires cyclique, oxazolidinone de formule générale III'a 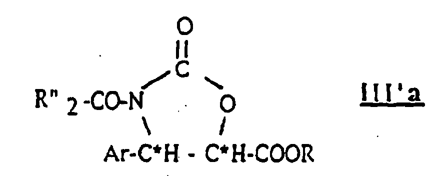 dans laquelle Ar et R sont définis précédemment, et R"₂ représente R'₂ défini précédemment, un radical alkoxy, de préférence t.butoxy, ou un radical alkyle linéaire ou ramifié comprenant au moins une insaturation, par exemple un radical 1-méthyl-1-propylène, et les dialkyl-acétals correspondants.

Les oxazolidinones de formule générale III'a sont obtenues tout d'abord en faisant réagir un dérivé de la β-aryl-isosérine de formule générale IIa avec un ester haloalkoxycarbonyle, en particulier 2,2,2-trichloroéthoxycarbonyle (TrOC), puis par cyclisation en présence d'une base organique forte, telle que le diazabicyclo-undécène (DBU). On obtient alors un dérivé d'oxazolidinone de formule générale IIIa 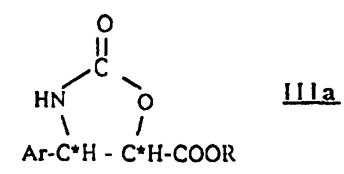 dans laquelle Ar et R sont définis précédemment.

Les dérivés de formule générale IIIa peuvent également être obtenus par synthèse directe, en faisant réagir les dérivés de β-aryl-glycidate de formule II'a avec de l'urée.

On obtient les dérivés acylés de formule générale III'a en introduisant le radical R"₂-CO- selon les techniques usuelles d'acylation, en présence d'un agent acylant approprié, par exemple un halogénure d'acyle de formule R"₂-CO-X, dans laquelle R"₂ est défini ci-dessus, et X représente un halogène, ou un anhydride de l'acide correspondant.

Les dialkylacétals sont obtenus selon les techniques usuelles de formation des acétals.

### 3.2 Ouverture de l'oxazoline de formule générale IIb

Par hydrolyse de l'oxazoline de formule générale llb en milieu acide, on obtient le dérivé de la β-aryl-isosérine de formule générale IIIb, 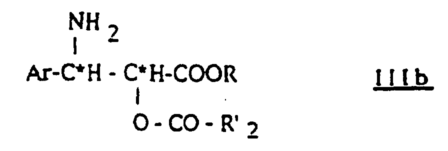 dans laquelle Ar, R et R'₂ sont définis précédemment.

D'une manière avantageuse, lorsque R'₂ représente un perhalogénoalkyle inférieur, tel que le trichlorométhyle, le radical R'₂ - CO - constitue un groupement protecteur de la fonction hydroxy.

On peut alors transformer ce précurseur de chaîne latérale de taxanes, en amide de formule générale III'b 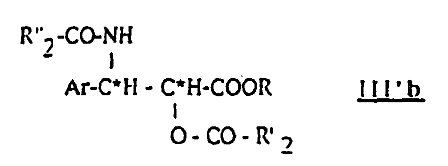 dans laquelle
Ar, R, R'₂ et R"₂ sont définis précédemment.

On peut donc obtenir indistinctement le précurseur de la chaine latérale du PACLITAXEL (R"₂ = phényle) ou du DOCETAXEL (R"₂ = t.butoxy).

### 4. Préparation du dérivé de l'acide cis-β-aryl-glycidique de formule I

Le dérivé de l'acide cis-β-aryl-glycidique de formule I peut être préparé selon les procédés usuels de l'état de la technique, ou par simple estérification de l'acide cis-β-aryl-glycidique avec l'alcool R-OH correspondant. Pour améliorer le rendement global de synthèse de précurseurs de chaine de taxanes, on prépare dans le procédé selon l'invention un dérivé cis-β-aryl-glycidate de formule générale I 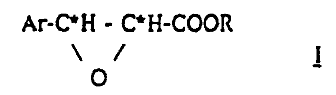 dans laquelle
Ar est défini précédemment et
R représente un énantiomère optiquement pur d'un radical hydro-carboné chiral de fort encombrement stérique,
en faisant réagir l'aldéhyde de formule

Ar-CHO

avec l'haloacétate de formule

X-CH₂-COOR

Ar, R étant définis précédemment et
X représentant un halogène, en particulier un chlore ou un brome.

D'une manière avantageuse, l'énantiomère optiquement pur d'un radical hydrocarboné chiral de fort encombrement stérique, est un cycloalkyle substitué par un ou plusieurs groupes alkyle, en particulier un cyclohexyle.

Il s'agit d'une réaction de Darzens par laquelle on obtient un mélange des deux diastéréoisomères, ester des acides cis-β-aryl-glycidiques, (2R,3R) et (2S,3S), et d'un énantiomère optiquement pur de l'alcool chiral R-OH, puisque la réaction de Darzens effectuée avec un haloacétate fortement encombré stériquement, conduit essentiellement à la forme cis du β-aryl-glycidate. D'une manière avantageuse, on choisira le radical hydrocarboné chiral de fort encombrement stérique de manière à ce qu'il permette la séparation physique des deux diastéréoisomères du milieu réactionnel, par exemple par cristallisation sélective, sans nécessiter une séparation stéréospécifique de l'énantiomère recherché en fin de réaction par les méthodes usuelles de cristallisation ou de chromatographie sur colonne chirale.

D'une manière avantageuse, R-OH représente le menthol, un des rares alcools chiraux de fort encombrement stérique, économique et disponible dans le commerce sous ses deux formes énantiomériques.

Dans le procédé de synthèse d'un précurseur de la chaîne latérale de taxanes, on cherche à préparer un cis-β-phényl-glycidate de configuration (2R,3R). Dans ce cas, on sélectionnera le radical hydro-carboné chiral de fort encombrement stérique R, de manière à ce que le diastéréoisomère du cis-β-phényl-glycidate de configuration (2R,3R) cristallise en premier du milieu réactionnel. Lorsque R-OH est le menthol, on emploie avantageusement le (+)-menthol.

La réaction de Darzens asymétrique s'effectue en présence d'une base, particulièrement un alcoolate alcalin tel que le tertiobutylate de potassium, ou un amidure, tel que le bis-triméthylsilylamidure de lithium dans un solvant approprié, en particulier un éther, tel que l'éther éthylique, à une température comprise entre - 78°C et 25°C. La réaction conduit à un mélange diastéréoisomérique constitué presque exclusivement des cis-glycidates pouvant atteindre un rendement supérieur à 95 %, voisin de 97 %. Le traitement du produit isolé dans un solvant approprié, en particulier un mélange méthanol-eau permet d'aboutir aisément à la séparation physique des diastéréoisomères requis.

Par cristallisation fractionnée (2 stades), on obtient un enrichissement rapide du diastéréoisomère recherché avec une pureté diastéréoisomérique supérieure à 99 %.

Ce dernier point est particulièrement important car il conditionne la pureté isomérique du taxane final, les diastéréoisomères indésirables présentant leur propre activité biologique, différente de celle du taxane recherché.

Il est remarquable de constater que l'emploi sélectif des deux énantiomères de l'ester menthylique permet d'accéder à l'aide du même procédé aux 2 diastéréoisomères précurseurs des deux énantiomères de l'acide glycidique.

En plus d'un rendement assez élevé en diastéréoisomère pur isolé (jusqu'à 45 %), la pureté diastéréoisomérique du produit majoritaire de la réaction, la facilité de mise en oeuvre de la réaction, la simplicité et la rapidité de la purification, le faible coût des réactants et catalyseurs, rendent d'accès facile et économique la synthèse industrielle de cet intermédiaire-clé dans la synthèse asymétrique des β-aminoacides.

Lorsque l'on utilise dans le procédé selon l'invention un dérivé de formule générale 1 obtenu par une réaction de Darzens asymétrique, on obtient alors les dérivés de formules générales IIa, II'a, IIb, IIIa, IIIb et III'b définis ci-dessus, pour lesquels R représente un énantiomère optiquement pur d'un radical hydrocarboné chiral de fort encombrement stérique, tel qu'un cycloalkyle substitué par un ou plusieurs groupes alkyle, en particulier un cyclohexyle, de préférence le menthyle, avantageusement le (+)-menthyle.

La présente invention concerne également ces dérivés, utiles comme intermédiaires dans la synthèse des chaînes latérales de taxanes.

Il convient de relever que le présent procédé constitue un accès très rapide aux oxazolines chirales substituées déjà décrites dans la littérature (WO 94/14787), en 3 étapes à partir de produits disponibles dans le commerce au lieu de 6 à 8.

### 5. Saponification menagée

On effectue une saponification ménagée des dérivés de formules générales IIa, II'a, IIb, IIIa, IIIb et III'b dans des conditions douces, de manière à libérer la fonction acide, tout en préservant la structure desdits dérivés, par exemple en présence d'un carbonate de métal alcalin dans un mélange méthanol/eau.

Après saponification ménagée, on obtient les dérivés de formules générales IIa, II'a, IIb, IIIa, IIIb et III'b définis précédemment, pour lesquels R représente un atome d'hydrogène, qui peuvent être employés directement dans l'hérnisynthèse de taxanes par couplage avec un dérivé approprié de la baccatine III .

### 6. Hémisynthèse de taxanes

### 6.1 Estérification

La présente invention concerne donc également un procédé d'hémisynthèse des taxanes de formule générale IV,

C-B IV

dans laquelle
C représente une chaîne latérale choisie parmi les radicaux de formules suivantes : 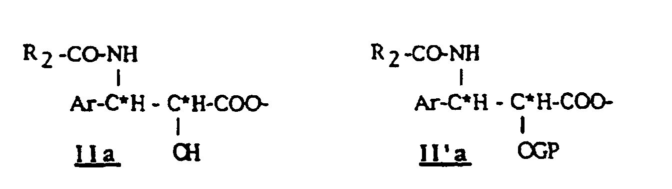 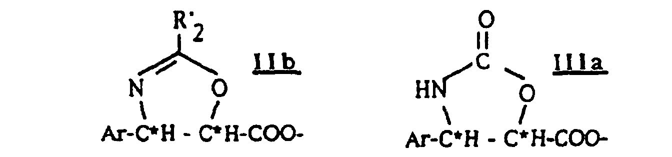 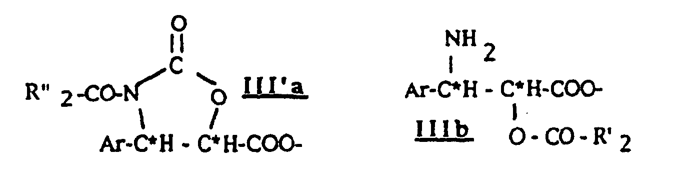 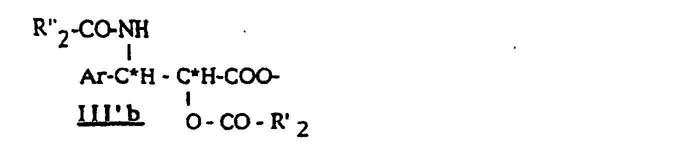 dans lesquelles Ar, R₂, R'₂, R"₂, R₃ et GP sont définis précédemment, et
B représente un radical dérivé de la baccatine III de formule générale V 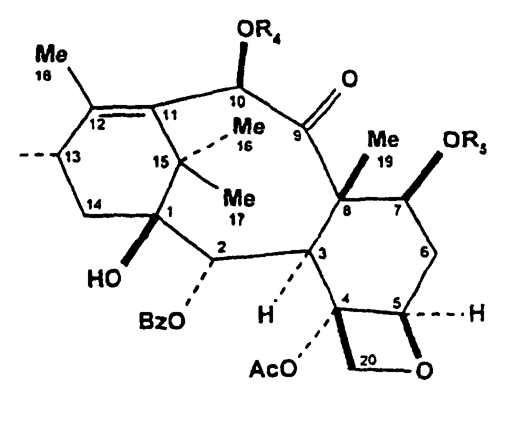 dans laquelle
Ac représente le radical acétyle,
Bz représente le radical benzoyle,
Me représente le radical méthyle,
R₄ représente un radical acétyle ou un groupe protecteur de la fonction hydroxy GP1, et
R₅ représente un groupe protecteur de la fonction hydroxy GP2,
par estérification d'un dérivé approprié de la baccatine III de formule générale V, porteur d'une fonction hydroxy en C-13, avec l'un des dérivés de formules générales IIa, II'a, llb, IIIa, III'a, IIIb et III'b définis précédemment, pour lesquels R représente un atome d'hydrogène, dans des conditions usuelles de préparation des taxanes telles que définies dans l'état de la technique (notamment : EP-0 253 738, EP-0 336 840, EP-0 336 841, EP-0 495 718, WO 92/09589, WO 94/07877, WO 94/07878, WO 94/07879, WO 94/10169, WO 94/12482, EP-0 400 971, EP-0 428 376, WO 94/14787).

Les groupements protecteurs GP1 et GP2 sont indépendamment l'un de l'autre des groupements usuels employés dans l'hémisynthèse des taxanes, tels que les trialkylsilyles (EP-0 336 840), ou le TrOC (EP-0 336 841).

GP1 et GP2 représentent également, indépendamment l'un de l'autre, des radicaux haloalkoxycarbonyle encombrés, linéaires ou ramifiés comprenant au moins un atome d'halogène. De manière avantageuse, il s'agit de radicaux dont le reste alkyle comprend entre 1 et 4 atomes de carbone, et 3 ou 4 atomes d'halogène, de préférence choisis parmi les radicaux 2,2,2-tribromoéthoxycarbonyle, 2,2,2,1-tétrachloroéthoxycarbonyle, 2,2,2-trichloro-*t*-butoxycarbonyle et trichlorométhoxycarbonyle, radicaux tous plus encombrés que l'haloalkoxycarbonyle (TrOC) utilisé jusqu'à présent pour protéger les taxanes en position 7.

GP1 et GP2 représentent également, indépendamment l'un de l'autre, des radicaux acyles dont le carbone en α de la fonction carbonyle porte au moins un atome d'oxygène.

Ces radicaux acyles sont notamment décrits dans la demande de brevet EP-0 445 021. Il s'agit avantageusement de radicaux alkoxy- ou aryloxyacétyle de formule

R₆-O-CH₂-CO-

dans laquelle R₆ représente un radical alkyle encombré stériquement, un radical cycloalkyle ou un radical aryle,
ou les radicaux arylidènedioxyacétyle de formule 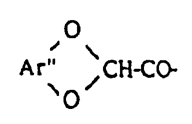 dans laquelle Ar" représente un radical arylidène.

Par alkyle encombré stériquement, on entend de préférence un radical alkyle linéaire ou ramifié en C₁-C₆ substitué par un ou plusieurs substituants encombrants choisi parmi les halogènes, les radicaux alkyle linéaires ou ramifiés en C₁-C₆, alkoxy linéaires ou ramifiés en C₁-C₆ ou cycloalkyle en C₃-C₆ ou aryle. Il s'agira par exemple d'un radical tertiobutyle ou triphénylméthyle.

Par cycloalkyle, on entend de préférence un radical cycloalkyle en C₃-C₆ éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle. D'une manière avantageuse, il s'agit d'un radical cyclohexyle, substitué par un ou plusieurs radicaux alkyle linéaires ou ramifiés en C₁-C₆, comme par exemple le menthyle, son racémique ou ses énantiomères et leurs mélanges en toutes proportions.

Par aryle, on entend de préférence un radical phényle, naphtyle, anthryle ou phénantryle, éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle, en particulier phényle. D'une manière préférentielle, il s'agit d'un radical phényle, éventuellement substitué par un ou deux substituants encombrants ci-dessus, en ortho- et ortho'- de la liaison éther.

Enfin, par arylidène, on entend de préférence un radical phénylène, naphtylène, anthrylène ou phénanthrylène, éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle, en particulier phényle.

GP1 et GP2 représentent également, indépendamment l'un de l'autre, un radical trialkylgermanyle ou forment ensemble un radical divalent de formule

-SiR₇-O-SiR₈-

dans laquelle
R₇ et R₈, indépendamment l'un de l'autre, représentent un radical alkyle encombré stériquement tel que défini ci-dessus, en particulier R₇ et R₈ représentent chacun un radical isopropyle.

### 6.2 Ouverture éventuelle

Lorsque C représente un radical de formule IIb ou IIIa, on effectue une ouverture du cycle oxazolinique pour obtenir un dérivé de taxanes de formule VI 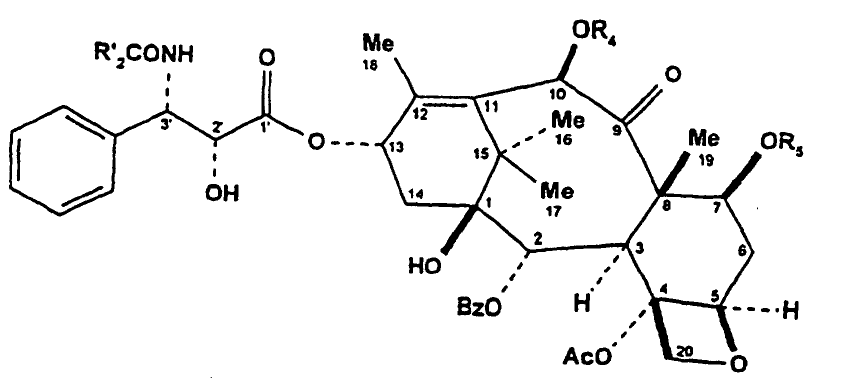 dans laquelle
Ac, Bz, Me, Ar, R₂, R₄ et R₅ sont définis précédemment.

L'ouverture des radicaux IIb, IIIa et III'a s'effectue généralement par hydrolyse en milieu acide ou basique. Pour le radical de formule IIb, cette ouverture peut être effectuée selon les méthodes décrites dans l'état de la technique (en particulier WO 94/14787) par hydrolyse en milieu acide, suivi d'un traitement en milieu basique pour obtenir le dérivé de formule générale VI.

### 6.3 Déprotection

Enfin, on effectue la déprotection des hydroxyles des dérivés de formule générale V ou VI, en remplaçant les groupes protecteurs de la fonction hydroxy GP (lorsque C représente le radical II'a), GP1 (lorsque R₄ est différent d'un acétyle), et GP2 par un atome d'hydrogène selon les techniques usuelles.

Pour les dérivés de formule générale V pour lesquels C représente un radical de formule IIb ou IIIa, et GP1 et/ou GP2 sont indépendamment l'un de l'autre des groupements usuels employés dans l'hémisynthèse des taxanes, tels que les trialkylsilyles, la déprotection s'effectue simultanément à l'ouverture décrite précédemment.

Lorsque GP1 et/ou GP2 sont des radicaux haloalkoxycarbonyle encombrés, la déprotection s'effectue selon les techniques usuelles décrites pour le TrOC, par action du zinc ou du zinc dopé avec des métaux lourds tels que le cuivre, dans un solvant organique, en particulier, dans l'acide acétique, le tétrahydrofurane ou l'alcool éthylique, avec ou sans eau.

Lorsque GP1 et/ou GP2 sont des radicaux acyles dont le carbone en a de la fonction carbonyle porte au moins un atome d'oxygène, la déprotection s'effectue en milieu basique par saponification dans le méthanol à basse température, avantageusement avec l'ammoniaque dans le méthanol à une température inférieure à 10°C, de préférence voisine de 0°C.

Pour le cas où C représente un radical de formule llb, l'ouverture de l'oxazoline s'effectue simultanément à la déprotection en milieu basique pour conduire en une étape au dérivé de taxane correspondant de formule générale VI, pour laquelle R₄ représente un radical acétyle ou un atome d'hydrogène, et R₅ représente un atome d'hydrogène, contrairement à l'ouverture en milieu acide décrite dans l'état de la technique qui nécessite une deuxième étape en milieu basique.

Les groupements protecteurs connus sont enlevés à l'aide de méthodes connues et la chaîne oxazolinique, quand elle était présente, déployée par hydrolyse, donnant des taxanes en tout point identiques aux taxanes de référence. A titre d'exemple, et pour montrer la validité de l'invention sans toutefois en limiter la portée, on peut obtenir du PACLITAXEL, du 10-désacétyltaxol, de la céphalomanine et du DOCETAXEL à partir des dérivés protégés correspondants.

Le déblocage des acyles dont le carbone en α de la fonction carbonyle porte au moins un atome d'oxygène, a d'abord été tenté dans les conditions classiquement considérées comme les plus douces, c'est-à-dire l'acétate de zinc en milieu méthanolique au reflux. Dans ce cas, la réaction étant complète en quelques heures (contre quelques jours pour les acétates), nous avons constamment isolé à côté du produit cherché, son épimère en C-7 résultant du classique équilibre de rétroaldolisation. Présumant que même dans les conditions neutres, voire légèrement acides, les principaux responsables étaient le méthanol et surtout la température, nous sommes revenus aux conditions standard de déblocage des acyles décrites par les premiers auteurs, par saponification en milieu basique dans l'éthanol à basse température. Dans ces conditions, aucune épimérisation notable n'a été constatée. A titre d'exemple, nous avons obtenu du PACLITAXEL, du 10-désacétyltaxol de la céphalomanine et du DOCETAXEL. en tout point identiques aux taxanes de référence, à partir des dérivés alkoxy- ou aryloxyacétylés correspondants.

Il convient enfin de noter que toutes les méthodes décrites auparavant, qui visent pourtant à améliorer le rendement global de l'hémisynthèse, consistent à synthétiser préalablement la chaîne phényl-isosérine, en vue de la transformer en l'une des structures cycliques citées plus haut (β-lactames, oxazolidines ou oxazolines). Donc, paradoxalement, les meilleures performances apparentes du couplage de ces structures cycliques ne font que compenser la baisse de rendement global provoquée par l'adjonction d'étapes de création de cycle à la séquence synthétique de la chaîne linéaire (soit un total de 9 étapes). Pour le procédé général de synthèse des taxanes selon l'invention, on obtient un produit tel que le PACLITAXEL en seulement 5 étapes :
- (2R,3R)-3-phénylglycidate de (1S,2R,5S)-(+)-menthyle
- (4S,5R)-2,4-Diphényl-4,5-dihydroxazole-5-carboxylate de ( 1S,2R, 5S)-(+)-menthyle
- saponification
- hémisynthèse (estérification)
- ouverture et déprotection.

La présente invention concerne enfin les intermédiaires de synthèse de formules générales IV, V et VI décrits précédemment, utiles dans la synthèse générale des taxanes objet de la présente invention.

D'une manière générale, par radical hydroxycarboné, on entend de préférence selon l'invention un radical hydrocarboné saturé ou insaturé, pouvant comprendre une ou plusieurs insaturations, tel qu'un alkyle linéaire ou ramifié, éventuellement insaturé, un cycloalkyle éventuellement insaturé, un aralkyle ou un aryle, chacun pouvant éventuellement être substitué par un ou plusieurs substituants, en particulier alkyle.

Par alkyle linéaire ou ramifié on entend de préférence selon l'invention un alkyle en C₁-C₆, en particulier choisi parmi les radicaux méthyle, éthyle, propyle, isopropyle, butyle et ses différents isomères ramifiés comme par exemple le terbutyle, pentyle et hexyle et leurs différents isomères ramifiés. Cette définition s'applique également aux restes alkyles des radicaux alkoxy ou aralkoxy.

Par cycloalkyle, on entend de préférence selon l'invention un cycloalkyle en C₃-C₆, en particulier choisi parmi les radicaux cyclopropyle, cyclobutyle, cyclopentyle ou cyclohexyle.

Par aryle on entend de préférence selon l'invention un radical aromatique ou hétéroaromatique, en particulier choisi parmi les radicaux phényle, naphtyle, anthryle, phénantryle, pyridyle, pyrimidyle, etc.

Enfin, par halogène on entend de préférence le chlore, le brome ou l'iode. Pour les radicaux haloalkoxycarbonyle, il s'agit de préférence de radicaux dont le reste alkyle comprend entre 1 et 4 atomes de carbone, et 3 ou 4 atomes d'halogène.

Le procédé général de synthèse de taxanes selon l'invention est rappelé sur le schéma 1 ci-après, pour R représentant le (+)-menthyle, et R₂ ou R'₂ représentant le phényle.

La dernière étape d'hémisynthèse des taxanes par le procédé selon l'invention est résumée sur les schémas 2 et 3 ci-après. Le schéma 2 résume une synthèse du PACLITAXEL à partir de dérivés de formule IV définis plus haut, pour lesquels C représente un radical de formules IIb ou III'a. Le schéma 3 résume la synthèse du 10-désacétyltaxol à partir d'un dérivé de formule IV pour lequel C représente un radical de formule IIb.

Bien entendu, les mêmes schémas de synthèse pourront être repris pour les autres définitions des substituants.

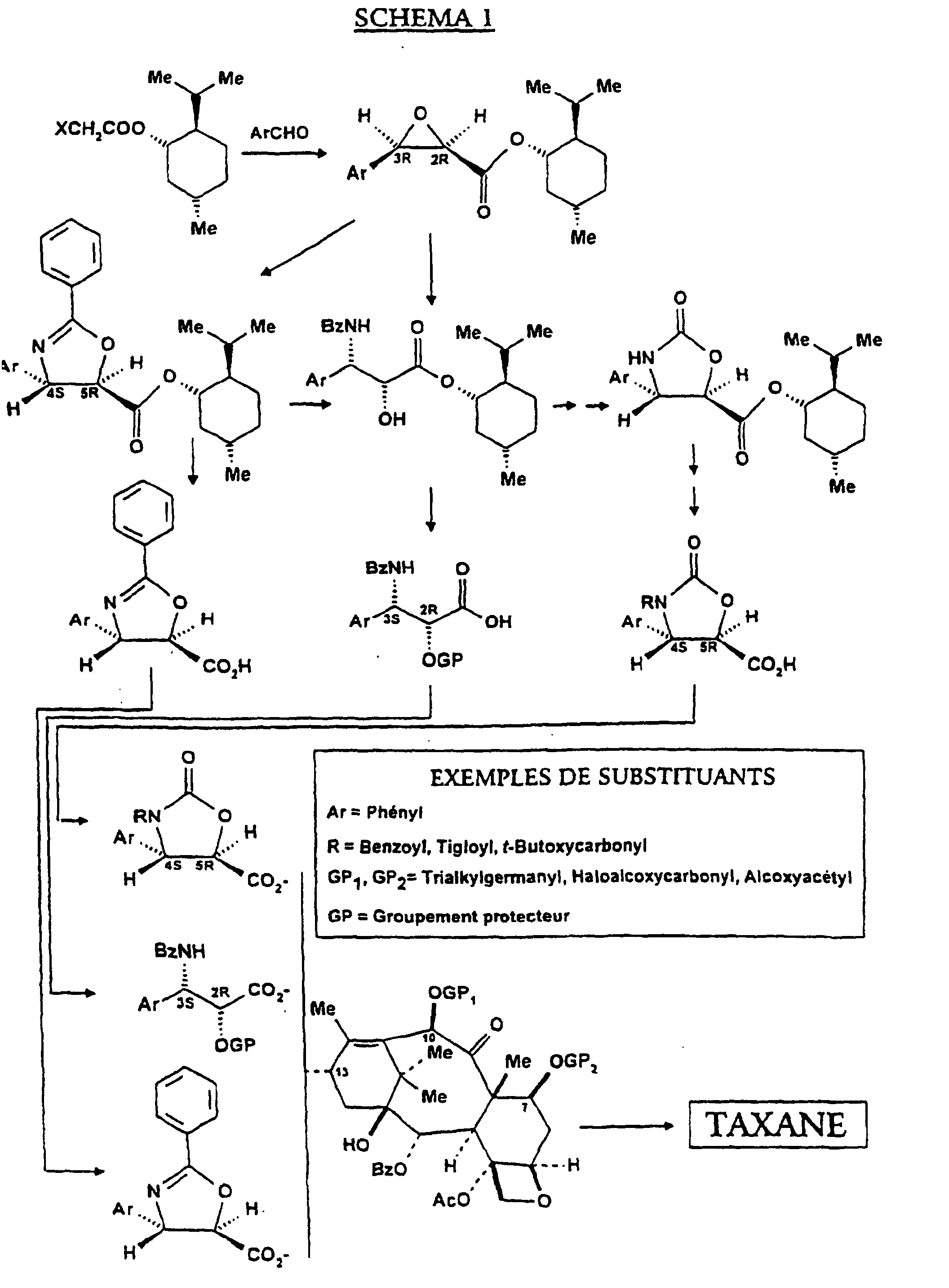

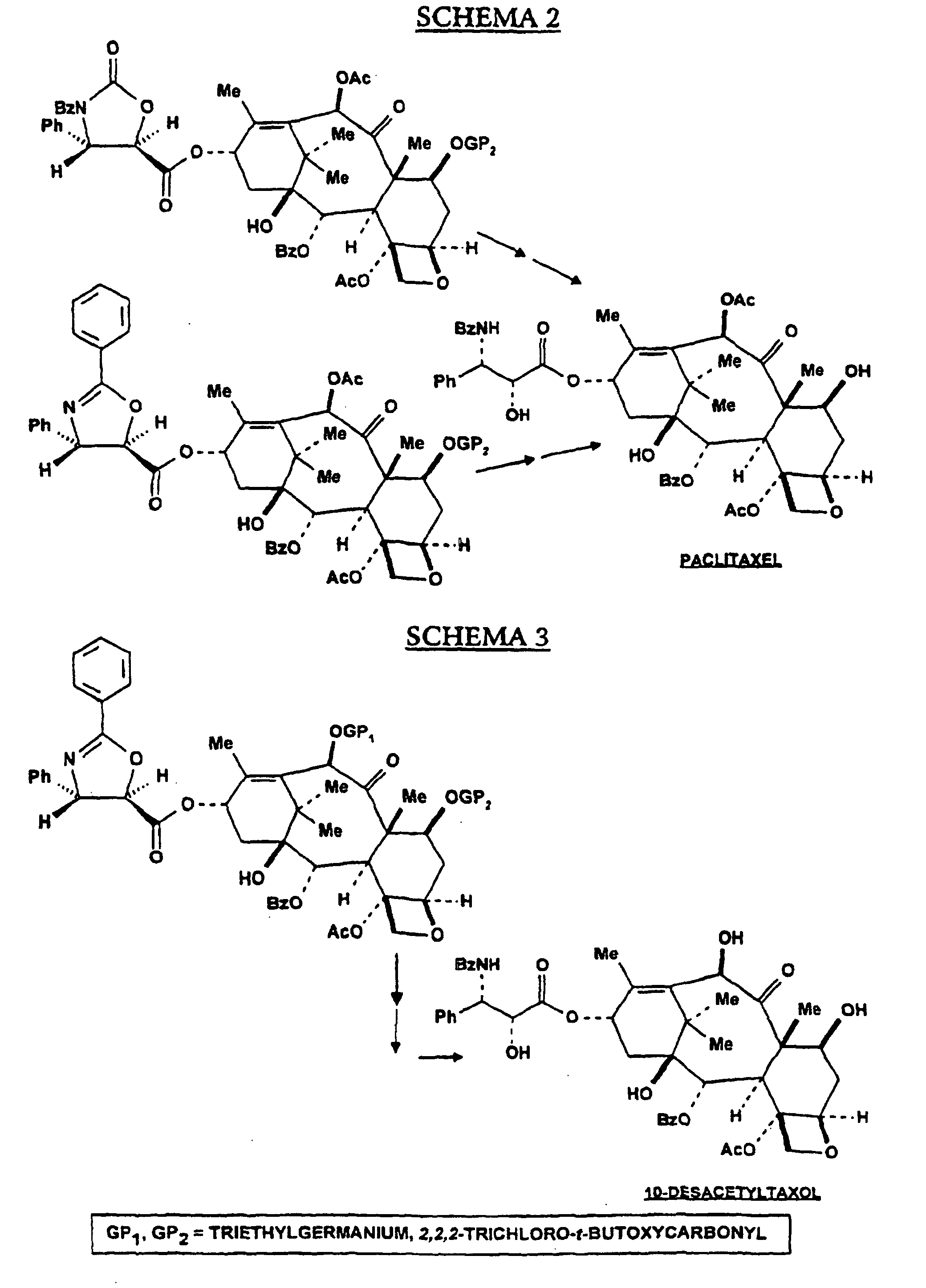

### PARTIE EXPERIMENTALE

### I. Précurseurs de chaine latérale de taxanes

### Exemple 1 :

Chloracétate de (1S,2R,5S)-(+)-menthyle

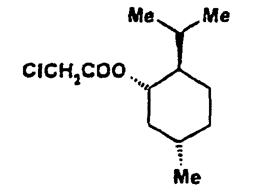

A une solution agitée à température ambiante de 100 g (0,640 mol) de (1S,2R,5S)-(+)-menthol dans 1 L de dichlorométhane sec, on ajoute 57mL (0,704 mol) de pyridine anhydre. Après quelques minutes d'agitation on additionne ensuite 56 mL (0,704 mol) de chlorure de chloracétyle, et on laisse se poursuivre la réaction pendant 30 min. Après contrôle par C.C.M, on ajoute 50 g de glace pilée et le milieu réactionnel est abandonné 1 h sous forte agitation. Après dilution avec 100 mL de dichlorométhane, la phase organique est lavée plusieurs fois avec une solution aqueuse saturée dechlorure de sodium (200 mL), séchée sur MgSO₄ puis concentrée sous pression réduite. Après purification du produit brut ainsi obtenu par chromatographie sur gel de silice (15-40 µm) (éluant: cyclohexane-acétate d'éthyle, 20/1) on obtient 146 g de chloracetate de (1S,2R,5S)-(+)-menthyle sous la forme d'un sirop.

Le composé obtenu présente les caractéristiques suivantes:
- RMN ¹H 400 MHz (CDCl₃) (δ ppm): 4,77 (1H, dt); 4,06 et 4,02 (2H, 2d, J = 13,6 Hz); 2,02 (1H, m, J = 11,8 Hz); 1,87 (1H, m, J = 7 et 2,6 Hz); 1,69 (2H, m); 1,50 (1H, m); 1,43 (1H, m, J = 11,7 et 3 Hz); 1,07 (1H, m); 1,02 (1H, q, J = 11,8 Hz); 0,92 et 0,90 (6H, 2d, J = 6,4 Hz); 0.89 (1H, m); 0,77 (3H, d, J = 7 Hz).

### Exemple 2 :

(2R,3R)-3-Phénylglycidate de (1S,2R,5S)-(+)-menthyle 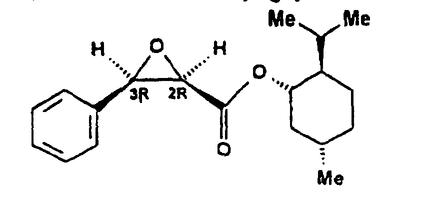 A une solution agitée à températureambiante de 152 g (0,653 mol) de chloracétate de (1S,2R,5S)-(+)-menthyle dans 600 mL d'éther éthylique anhydre, on ajoute 69 mL (0,686 mole) de benzaldéhyde. Après quelques minutes d'agitation, la solution est refroidie à -78°C sous atmosphère inerte, on ajoute ensuite en 2 h une suspension de 85 g (0,718 mol) de *tert*-butylate de potassium dans 400 mL d'éther éthylique anhydre, et on laisse revenir le milieu réactionnel à température ambiante. Après contrôle par C.C.M, la partie organique est diluée avec 200 mL de dichlorométhane, lavée plusieurs fois avec une solution saturée de chlorure de sodium, séchée sur MgSO₄ et concentrée sous pression réduite. On obtient ainsi 200 g d'un produit brut à l'état de sirop, contenant quatre diastéréoisomères (dont deux *cis* et deux *trans*), et que l'on soumet tel que à une cristallisation fractionnée.

Dans un premier temps, la solution du produit brut dans 2 L de méthanol portée à 60°C, à laquelle on ajoute progressivement 700 mL d'eau osmosée, est abandonnée 16 h à température ambiante et à l'écart des vibrations. Une phase solide inférieure de couleur jaune, riche en isomères *trans*, est écartée, et les cristaux blancs de la phase supérieure, riches en isomères *cis*, sont séparés par filtration. Les cristaux ainsi obtenus sont remis en solution dans 2 L de méthanol porté à 60°C, avec addition de 500 mL d'eau osmosée jusqu'à obtention d'un trouble persistant, et abandon 16 h à température ambiante. Trois cristallisations supplémentaires effectuées selon le même procédé, mais avec des volumes réduits en méthanol (1 L) ct en eau (200 mL), sont nécessaires pour obtenir 23 g de (2R,3R)-3-phénylglycidate de (1S,2R,5S)-(+)-menthyle à l'état cristallisé avec une pureté HPLC > 99 % (Rdt = 12 %).

Le composé obtenu présente les caractéristiques suivantes:
- F = 104°C
- RMN ¹H 400 MHz (CDCl₃) (δ ppm): 7,40 (2H, dd, J = 7,8 Hz et 1,7 Hz); 7,32 (3H, m); 4,58 (1H, dt, J = 10,9 Hz et 4,2 Hz); 4,26 (1H. d, J = 4,6 Hz); 3,83 (1H, d, J = 4,8 Hz); 1,6 à 0,85 (9H, m); 0,78 (3H, d, J = 7 Hz); 0,75 (3H, d, J = 6,4 Hz); 0,62 (3H, d, J = 6,9 Hz).
*Diffraction des rayons X d'un monocrystal de (2R*,*3R)-3-phénylglycidate de (1S*,*2R*,*5S)-(+)-menthyle en vue de la détermination indirecte de la configuration absolue:*

Le monocrystal a été obtenu à partir d'une suspension crystalline résultant de l'addition à chaud du non-solvant (eau) sur une solution hémisaturée du glycidate dans le méthanol. Par refroidissement lent, la solution a laissé déposerde fines aiguilles de pureté 99,95 % (CLHP), qui ont été conservées humectées jusqu'à la sélection finale.

L'échantillon sélectionné (aiguille fine de dimensions 0.12∗0.12∗0.40 mm) a été étudié sur un diffractomètre automatique CAD4 ENRAF-NONIUS (radiation du molybdène avec monochromateur au graphite). les paramètres de la maille ont été obtenus par affinement d'un ensemble de 25 réflexions à angle thêta élevé. la collection de données (2θₘₐₓ = 50°, balayage ω/2θ = 1, tₘₐₓ = 60 s, domaine HKL: H 0,6 K 0,14 L 0,28, contrôlesd'intensité sans dérive significative (0.1 %)) a fourni 1888 réflexions dont 1037 avec I>1.5σ(I).
C₁₉H₂₆O₃: Mr = 302.42, orthorhombique, P2₁2₁2₁, a = 5.709(11). b = 12,908(4), c = 24.433(8) Å, V = 1801(5) Å⁻³, Z = 4, Dₓ = 1,116 Mg.m⁻³, λ(MoKα) = 0.70926 Å, µ = 0.69 cm⁻¹, F(000) = 656, T = 294 K, R final = 0.072 pour 1037 observations.

Après corrections de Lorenz et corrections de polarisation, la structure a été résolue à l'aide des Méthodes Directes qui permettent de localiser la majorité des atomes non hydrogènes de la molécule, les atomes restant étant localisés par différences de Fourier et mises à l'échelle successives. Après affinement isotrope (R = 0.125) puis anisotrope (R = 0.095), la plupart des atomes d'hydrogène sont localisés à l'aide d'une différence de Fourier (entre 0.39 et 0.14 eÅ⁻³), les autres étant positionnés par le calcul. la structure complète a été affinée par matrice entière (x, y, z, βᵢⱼ pour C et O, x, y, z pour H; 200 variables et 1037 observations; w = l/σ(Fₒ)²=[σ²(I) + (0.04Fₒ²)²]^{-1/2}) conduisant à R = 0.080, R_{w} = 0.072 et S_{w} = 1.521 (résidu Δ ρ ≤ 0.21 eÅ⁻³).

Les facteurs de diffusion sont tirés des Tables Internationales de cristallographie [International Tables for Xray Cristallography (1974). Vol.IV.Birmingham: Kynoch Press. (Présent distributeur D.Reidel,Dordrecht)]. Les calculs ont été réalisés sur Hewlett Packard 9000-710 pour la détermination de la structure [SHELDRICK, G. M. (1985). Crystallographic Computing 3: Data Collection, Structure Determination, Proteins and Databases edited by G. M. Sheldrick, C. Krüger and R. Goddard. Oxford: Clarendron Press] et sur Digital Micro VAX 3100 pour les autres calculs avec le jeu de programmes MOLEN [FAIR, C.K. (1990). MOLEN. An Interactive Intelligent System for Crystal Structure Analysis. Enraf-Nonius, Delft, The Netherlands].

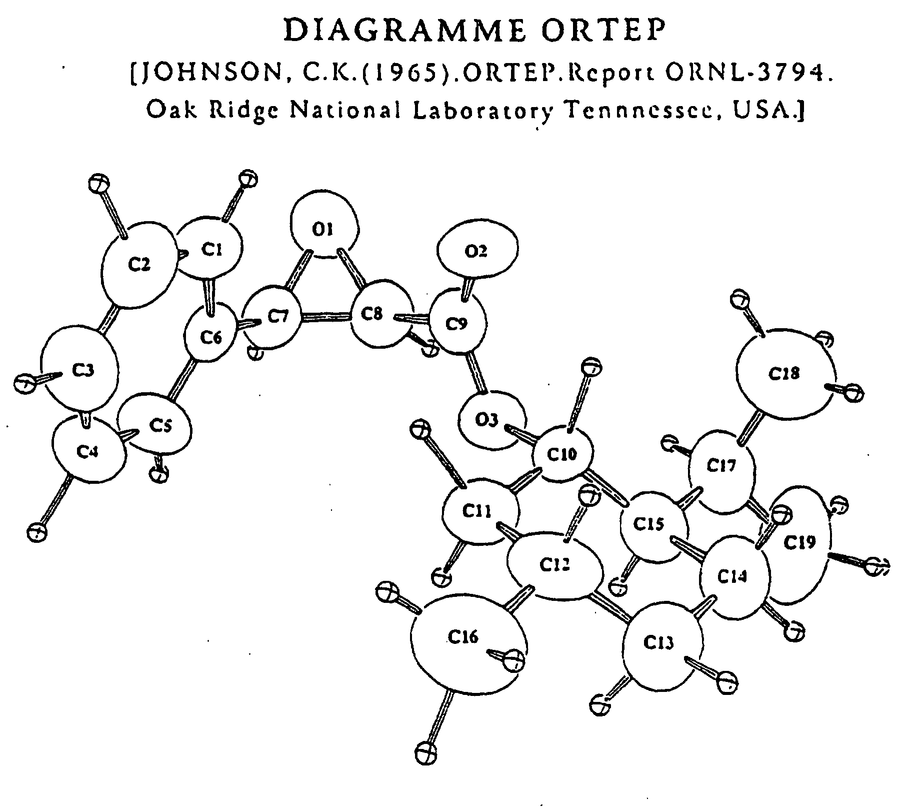

Un échantillon (2R,3R)-3-phényl-glycidate de (1S,2R,5S)-(+)-menthyle, par traitement au méthylate de sodium dans le méthanol, a permis d'obtenir le phénylglycidate de méthyle correspondant dont les caractéristiques sont les suivantes:
- [α]_{D}²⁸ = + 12 (c = 1,15; chloroforme)
- RMN ¹H 400 MHz (CDCl₃) (δ ppm): 7,40 (2H, d, J = 8 Hz); 7,32 (3H, m); 4,26 (1H, d, J = 4,6 Hz); 3,84 (1H, d, J = 4,6 Hz); 3,55 (3H, s).

### Exemple 3 :

(4S,5R)-2,4-Diphényl-4,5-dihydrooxazole-5-carboxylate de (1S,2R,5S)-(+)-menthyle

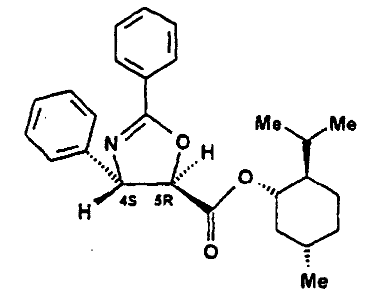

A une solution agitée sous atmosphère inerte à -65°C de 30 g (0,0993 mol) de (2R,3R)-3-phénylglycidate de (1S,2R,5S)-(+)-menthyle et 305 mL (2,98 mol) de benzonitriledans 1,5 L de dichlorométhane anhydre, on ajoute 15 mL (0,109 mol) d'une solution à 54 % d'acide tétrafluoroborique dans l'éther en 10 min. On laisse la réaction se poursuivre à -65°C pendant I h et, après contrôle par C.C.M, on additionne 300 mL d'une solution aqueuse saturée d'hydrogénocarbonate de sodium et on laisse revenir à température ambiante avec agitation. Après extraction de la phase aqueuse avec du dichlorométhane (2 x 200 mL), les phases organiques réunies sont lavées avec une solution saturée de chlorure desodium (200 mL), avec de l'eau (50 mL) et séchée sur MgSO₄. Après concentration sous pression réduite et élimination du benzonitrile résiduel sous vide poussé à 50°C, le produit brut obtenu est purifié par chromatographic sur gel de silice (15-40 µm) (éluant: cyclohexane-acétate d'éthyle, 20/1).

On isole ainsi 32 g de (4S,5R)-2,4-diphényl-4,5-dihydrooxazole-5-carboxylate de (1S,2R,5R)-(+)-menthyle sous la forme d'un sirop incolore (Rdt = 80 %) et qui présente les caractéristiques suivantes:
- RMN ¹H 400 MHz (CDCl₃) (δ ppm): 8,10 (2H, d, J = 7,1 Hz); 7,54 (1H, t, J = 7,4 Hz); 7,46 (2H, t, J = 7,4 Hz); 7,34 (5H, m); 5.40 (1H, d, J = 6,4 Hz); 4.88 (1H, d, J = 6,4 Hz); 4,85 (1H, dt, J = 10,9 et 4,4 Hz); 2,09 (1H, m); 1,84 (1H, m, J = 7 et 2,7 Hz); 1,71 (1H, m); 1,69 (1H, m); 0,94 (3H, d, J = 6,5 Hz); 0,9 (1H, m); 0,85 (3H, d, J = 7 Hz); 0,77 (3H, d, J = 7 Hz).

### Exemple 4 :

Acide (4S,5R)-2,4-diphényl-4,5-dihydrooxazole-5-carboxylique

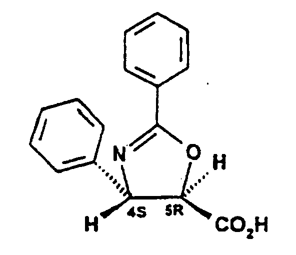

A une solution agitée à tcmpérature ambiante de 3,5 g (8,64 mmol) de (4S,5R)-2,4-diphényl-4,5-dihydrooxazole-5-carboxylate de (1S,2R,5S)-(+)-menthyle dans le méthanol (70 mL), on ajoute 25 mL d'une solution de 6 g (43,2 mmol) de carbonate de potassium dans l'eau osmosée, et on laisse la réaction se poursuivre pendant 16 h à température ambiante. Après contrôle par C.C.M, le milieu réactionnel est concentré sous pression réduite. La phase aqueuse ainsi obtenue est lavée avec du dichlorométhane (3 x 100 mL), acidifiée à pH 2 par addition lente de 20 mL d'une solution aqueuse d'HCl 1M, et extraite avec de l'acétate d'éthyle (3 x 100 mL). Les phases organiques d'extraction réunies sont séchées (MgSO₄) et concentrées sous pression réduite.

On obtient ainsi 2,26 g d'acide (4S,5R)-2,4-diphényl-4,5-dihydrooxazole-5-carboxylique sous la forme d'une poudre blanche (Rdt = 98 %) et qui présente les caractéristiques suivantes:
- [α]_{D}²² = + 27,7 (c = 0,99; CH₂Cl₂-MeOH, 1/1)
- F = 201-202 °C
- RMN ¹H 400 MHz (DMSO-d₆) (δ ppm): 7,99 (2H, d, J = 7,3 Hz); 7,64 (1H, t, J = 7,4 Hz); 7,55 (2H, t, J = 7,7 Hz); 7,36 (5H, m); 5,40 (1H, d, J = 6,3 Hz); 4,99 (1H, d, J = 6,4 Hz).

### Exemple 5 :

(2R,3S)-*N*-Benzoyl-3-phénylisosérinate de (1S,2R,5S)-(+)-menthyle

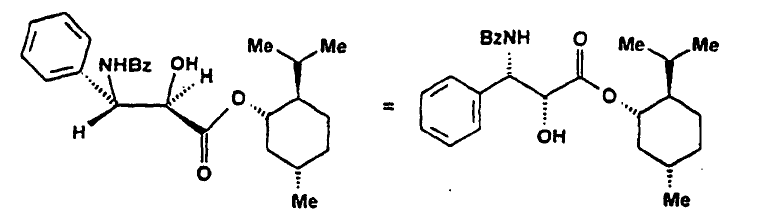

A une solution agitée à température ambiante de 1 g (2,47 mmol) de (4S,5R)-2,4-diphényl-4,5-dihydrooxazole-5-carboxylate de (1S,2R,5S)-(+)-menthyle dans un mélange de méthanol (15 mL) et de tétrahydrofurane (15 mL), on ajoute 15 mL d'une solution aqueuse d'HCl 1M. Le mileu réactionnel est porté 1 h à reflux, et après contrôle par C.C.M et retour à température ambiante une solution aqueuse saturée d'hydrogénocarbonate de sodium (45 mL) est progressivement ajoutée jusqu'à obtention d'un pH basique. Après 48 h d'agitation à température ambiante, la phase aqueuse obtenue après concentration sous pression réduite est extraite au dichlorométhane (100 mL). La phase aqueuse est lavée avec une solution saturée de chlorure de sodium (2 x 50 mL), séchée sur MgSO₄, concentrée sous pression réduite, et le résidu obtenu est chromatographié sur gel de silice (15-40 µm) (éluant: dichlorométhane-méthanol, 95/05).

On isole ainsi 0.835 g de (2R,3S)-*N*-Benzoyl-3-phénylisosérinate de (1S,2R,5S)-(+)-menthyle sous la forme d'un solide blanc (Rdt = 80 %) et qui présente les caractéristiques suivantes:
- RMN ¹H 400 MHz (CDCl3) (δ ppm): 7,77 (2H, d, J = 7,2 Hz); 7,51(1H,t, J= 7,3 Hz); 7,45 (4H, m); 7.36 (2H, t, J= 7,2 Hz); 7,29 (1H, t, J= 7,2 Hz); 7,04 (1H, d, J = 9,2 Hz); 5,78 (1H,dd, J = 9,2 et 2,1 Hz); 4,79 (1H, dt, J = 10,9 et 4,4 Hz); 4,63 (1H, s large); 3,35 (1H, s large); 1,81 (2H, m); 1,67 (3H, m); 1,5 à 1,36 (2H, m); 1,09 à 0,91 (2H, m); 0,89 (3H, d, J= 6,9 Hz); 0,77 (3H, d, J= 6,5 Hz); 0,74 (3H, d, J= 6,9 Hz).

### Exemple 6 :

(2R,3S)-*N*-Benzoyl-*O*-triéthylsilyl-3-phénylisosérinate de (1S,2R,5S)-(+)-menthyle

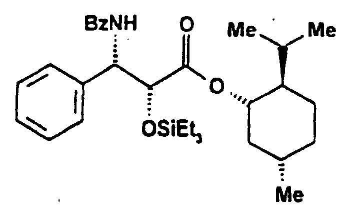

A une solution de 0.8 g (1,89 mmol) de (2R,3S)-*N*-benzoyl-3-phénylisosérinate de (1S,2R,5S)-(+)-menthyle dans 10 mL de dichlorométhane anhydre on ajoute 0.255 g (2,08 mmol) de 4-diméthylaminopyridine. Après quelques minutes d'agitation à température ambiante, on ajoute 477 µL (2,84 mmol) de chlorure de triéthylsilyle en 5 min. Après 1 h d'agitation à température ambiante et contrôle par C.C.M, le milieu réactionnel est dilué avec 100 mL de dichlorométhane. La phase organique est lavée avec une solution aqueuse saturée d'hydrogénocarbonate de sodium (2 x 20 mL), avec une solution saturée de chlorure de sodium (50 mL), séchée sur MgSO₄ ct concentrée sous pression réduite. Après purification du résidu obtenu par chromatographic sur gel de silice (15-40 µm) (éluant: cyclohexane-acétate d'éthyle, 10/1) on obtient 0,74 g de (2R,3S)-*N*-benzoyl-*O*-triéthylsilyl-3-phénylisosérinate de (1S,2R,5S)-(+)-menthyle sous la forme d'un sirop incolore (Rdt = 75 %).

Le composé obtenu présente les caractéristiques suivantes:
- RMN ¹H 400 MHz (CDCl₃) (δ ppm): 7,82 (2H, d, J= 7 Hz); 7,52 (1H, t, J= 7,4 Hz); 7,45 (2H, t, J= 7 Hz); 7,37 (2H, d, J= 7,2 Hz); 7,32 (2H, t, J = 7,2 Hz); 7,26 (2H, m); 5,60 (1H, dd); 4,73 (1H, dt, J= 11 et 4,3 Hz); 1,88 à 1,67 (m); 1,44 (2H, m); 1,06 à 0,87 (m); 0,80 (m); 0,67 (3H, d, J= 7 Hz); 0,62 à 0,34 (m).

### Exemple 7 :

(2R,3S)-*N*-Benzoyl-*O*-triéthylsilyl-3-phénylisosérine

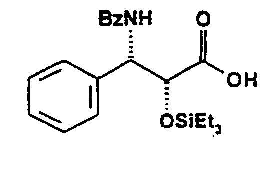

A une solution agitée à température ambiante de 0,5 g (0,931 mmol) de (2R,3S)-*N*-benzoyl-*O*-triéthylsilyl-3-phénylisosérinate de (1S,2R,5S)-(+)-menthyle dans 15 mL de méthanol, on ajoute une solution de 0.644 g (4,655 mmol) de carbonate de sodium dans 10 mL d'eau osmosée. Après agitation 16 h à température ambiante et contrôle par C.C.M, le milieu réactionnel est concentré sous pression réduite, et la phase aqueuse résiduelle est lavée au dichlorométhane (3 x 50 mL) puis acidifiée à pH 2 par addition lente d'une solution aqueuse d'HCl 1M (10 mL). La phase aqueuse est extraite avec de l'acétate d'éthyle (3 x 50 mL) et les phases organiques réunies sont séchées sur MgSO₄ et concentrées sous pression réduite.

On obtient 0,320 g de (2R,3S)-*N*-benzoyl-*O*-triéthylsilyl-3-phénylisosérine sous la forme d'une poudre blanche (Rdₜ = 90 %) et qui présente les caractéristiques suivantes:
- RMN ¹H 400 MHz (DMSO-d₆) (δ ppm): 8,46 (1H,d, J = 9,3 Hz); 7,82 (2H, d, J = 7,1 Hz); 7,54 (1H, t, J = 7,2 Hz); 7,47 (4H, m); 7,32 (2H, t); 7,36 (1H, t); 5,44 (1H, dd, J = 9,2 et 5,5 Hz); 4,64 (1H, d, J = 5,6 Hz); 0,77 (9H, m); 0,45 (6H, m).

### Exemple 8 :

(2R,3S)-*N*-Benzoyl-*O*-(2,2,2-trichloroéthoxy)carbonyl-3-phénylisosérinate de (1S,2R,5S)-(+)-menthyle

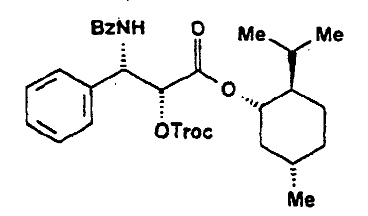

A une solution agitée à température ambiante sous atmosphère inerte de 1,38 g (3,3 mmol) de (2R,3S)-*N*-benzoyl-3-phénylisosérinate de (1S,2R,5S)-(+)-menthyle dans 30 mL de dichlorométhane anhydre on ajoute 480 mg (3,96 mmol) de 4-diméthylaminopyridine. Après 10 min d'agitation, on ajoute 540 µL (3,96 mmol) de chlorure de 2,2,2-trichloroéthoxycarbonyle en 5 min. Après 2 h d'agitation à température ambiante et contrôle par C.C.M., la phase organique est lavée avec une solution saturée d'hydrogénocarbonate de sodium (2 x 10 mL), avec une solution saturée de chlorure de sodium (10 mL). séchée sur MgSO₄ et concentrée sous pression réduite. Après purification du résidu obtenu par chromatographie sur gel de silice (15-40 µm) (éluant: cyclohexane-acétate d'éthyle 5/1) on obtient 1,60 g de (2R,3S)-*N*-benzoyl-*O*-(2,2,2-trichloroéthoxy)carbonyl-3-phénylisosérinate de (1S,2R,5S)-(+)-menthyle sous la forme d'un sirop incolore (Rdt = 82%).

Le composé obtenu présente les caractéristiques suivantes:
- RMN ¹H 400 MHz (CDCl₃)(δ ppm): 7,82 (2H, d, J = 7,4 Hz); 7,53 (1H, t, J = 7,4 Hz); 7,44 (4H, m); 7,35 (2H, t, J = 7 Hz); 7,29 (1H, t, J = 7 Hz); 7,09 (1H, d, J = 9,3 Hz); 6,0 (1H, dd, J = 9,3 et 2,5 Hz); 5,45 (1H, d, J = 2,6 Hz); 4,78 et 4,72 (2H, 2d, J = 11,9 Hz); 4,77 (1H, m); 1,85 (1H, m) 1,79 (1H, m); 1,65 (2H, m); 1,43 (1H, m); 1,02 (1H, m); 0,96 (1H, m); 0,86 (1H, m); 0,83 (3H, d, J = 7 Hz); 0,78 (3H, d, J = 6,5 Hz); 0,68 (3H, d, J = 6,9 Hz).

### Exemple 9 :

(4S,5R)-4-Phényloxazolidin-2-one-5-carboxylate de (1S,2R,5S)-(+)-menthyle

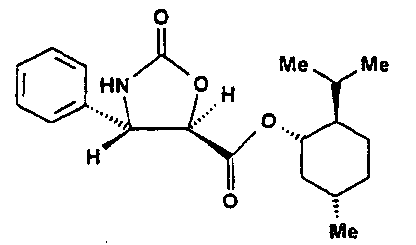

A une solution agitée sous atmosphère inerte à température ambiante de 3,96 g (6,62 mmol) de (2R,3S)-*N*-benzoyl-*O*-(2,2,2-trichloroéthoxy)-carbonyl-3-phénylisosérinate de (1S,2R,5S)-(+)-menthyle dans 30 mL de dichlorométhane anhydre, on ajoute 1 mL (7,28 mmol) de 1,8-diazabicylo [5,4,0] undec-7-ène. Après 30 min d'agitation à température ambiante, la phase organique est lavée avec 10 mL d'une solution saturée de chlorure de sodium, séchée sur MgSO₄ et concentrée sous pression réduite. Après purification du résidu par chromatographie sur gel de silice (15-40 µm) (éluant: cyclohexane - acétate d'éthyle 7/3) on obtient 2,18 g du composé cité en titre sous la forme d'un sirop jaune (Rdt = 95 %).

Le composé obtenu présente les caractéristiques suivantes:
- RMN ¹H 400 MHz (CDCl₃)(δ ppm): 7,40 (5H, m): 6,09 (1H, s): 4,93 (1H, d, J = 5,3 Hz); 4,86 (1H, dt, J = 11 et 4.4 Hz); 4,73 (1H, d. J = 5,4 Hz); 2,05 (1H, m); 1,81 (1H, m); 1,71 (2H, m); 1,54 à 1,41 (3H, m); 1,07 (2H, m); 0,94 (3H, d, J = 6,5 Hz); 0,88 (3H, d, J = 7 Hz); 0,77 (3H, d, J = 7 Hz).

### Exemple 10 :

(4S,5R)-*N*-^{t}Butoxycarbonyl-4-phényloxazolidin-2-one-5-carboxylate de (1S,2R,5S)-(+)-menthyle

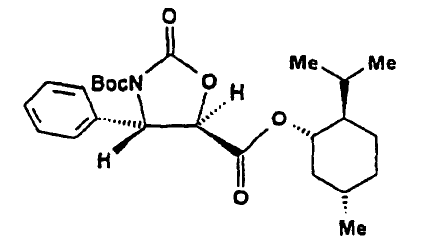

A une solution agitée à -40°C sous atmosphère inerte de 1,91 g (5,52 mmol) de (4S,5R)-4-phényloxazolidin-3-one-5-carboxylate de (1S,2R,5S)-(+)-menthyle dans 20 mL de tétrahydrofurane anhydre, on additionne 3,8 mL (6,07 mmol) d'une solution 1,6 M de *n*-butyl lithium dans l'hexane. Après 10 min d'agitation à - 40°C, on ajoute une solution de 1,81 g (8,28 mmol) d'anhydride ^{t}butoxycarbonique en solution dans 5 mL de tétrahydrofurane, et on laisse le milieu réactionnel revenir à température ambiante en 15 min. Après dilution avec 50 mL de dichlorométhane et lavage avec une solution aqueuse d'HCl 2 % jusqu'à obtention d'un pH = 5, la phase organique est séchée (MgSO₄) et concentrée sous pression réduite. Après purification du produit brut par chromatographie surgel de silice (15-40 µm) (éluant: cyclohexane-acétate d'éthyle, 5/1) on obtient 2,12 g du composé cité en titre sous la forme d'un sirop incolore (Rdt = 86 %).

Le composé ainsi obtenu présente les caractéristiques suivantes:
- RMN ¹H 400 MHz (CDCl₃) (δ ppm): 7,45 à 7,26 (5H, m); 5,19 (1H, d, J = 3,7 Hz); 4,86 (1H, dt, J = 10,9 et 4,5 Hz); 4,66 (1H, d, J = 3,7 Hz); 2,05 (1H, m); 1,79 (1H, m); 1,73 (2H, m); 1,62 à 1,24 (3H, m); 1,33 (9H, s); 1,11 (2H, m); 0,94 (3H, d, J = 6,5 Hz) et (1H, m); 0,89 (3 H, d, J = 7Hz); 0,77 (3H, d, J = 7 Hz).

### Exemple 11 :

(4S,5R)-3-*N*-Benzoyl-4-phényloxazolidin-3-one-5-carboxylate de (1S,2R,5S)-(+)-menthyle

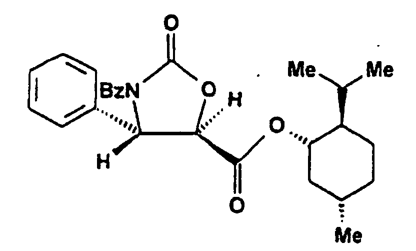

A une solution agitée sous atmosphère inerte à température ambiante de 500 mg (1,45 mmol) de (4S,5R)-4-phényloxazolidin-3-one-5-carboxylate de (1S,2R,5S)-(+)-menthyle et 176 mg (1,16 mmol) de 4-pyrrolidinopyridine dans 7 mL de dichlorométhane anhydre, on ajoute 0,25 mL (2,17 mmol) de chlorure de benzoyle. Après 3 h d'agitation à 50°C, le milieu réactionnel est ramené à température ambiante et dilué avec 20 mL de dichlorométhane. La phase organique est lavée avec 10 mL d'une solution saturée de chlorure de sodium, séchée sur MgSO₄ et concentrée sous pression réduite. Après purification du produit brut par chromatographie sur gel de silice (15-40 µm)(éluant: cyclohexane - acétate d'éthyle5/1) on obtient 300 mg du composé cité en titre sous la forme d'un sirop incolore (Rdt = 46 %).

Le composé ainsi obtenu présente les caractéristiques suivantes:
- RMN ¹H 400 MHz (CDCl₃)(δ ppm): 8,16 (2H, d, J = 7,1 Hz); 7,68 (1H, t); 7,53 (4H, m); 7,43 (3H, m); 5,57 (1H, d, J = 4,4 Hz); 4,90 (1H, dt, J = 10,9 et 4,4 Hz); 4,85 (1H, d, J = 4,3 Hz); 2,07 (1H, m); 1,80 (1H, m); 1,72 (2H, m); 1,47 (3H, m); 1,09 (2H, m); 0,95 (3H, d, J = 6,5 Hz); 0,88 (3H, d, J = 7 Hz); 0,78 (3H, d, J = 7 Hz).

### Exemple 12 :

Acide (4S,5R)-3-*N*-benzoyl-4-phényloxazolidin-3-one-5-carboxylique

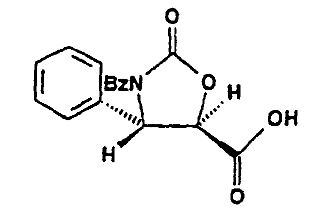

A un mélange agité à température ambiante de 120 mg (0,266 mmol) de (4S,5R)-3-*N*-benzoyl-4-phényloxazolidin-3-one-5-carboxylate de (1S,2R,5S)-(+)-menthyle dans 2 mL de méthanol, on ajoute une solution de 75 mg (0,543 mmol) de carbonate de potassium dans 1 mL d'eau. Après 30 min d'agitation, le milieu réactionnel est dilué avec 10 mL d'eau et la phase aqueuse est lavée avec 5 mL de dichlorométhane. Après acidification à pH = 4 au moyen d'HCl 1M, la phase aqueuse résiduelle est extraite à l'acétate d'éthyle (3 x 10 mL). Les phases organiques réunies sont lavées avec 5 mL d'une solution saturée de chlorure de sodium, séchées sur MgSO₄ et concentrées sous pression réduite.

On obtient 40 mg d'acide (4S,5R)-3-*N*-Benzoyl-4-phényloxazolidin-3-one-5-carboxylique sous la forme d'une poudre blanche (Rdt = 52 %), et qui présente les caractéristiques suivantes:
- RMN ¹H 400 MHz (DMSO · d₆)(δ ppm): 12.98 (1H, s large); 7,95 (2H, d, J = 7,1 Hz); 7.63 (1H, t, J = 7,4 Hz); 7,50 (2H, t, J = 7,5 Hz); 7,42 (2H, m); 7,37 (3H, m); 4,90(1H, d, J = 5 Hz); 4,77 (1H, d, J = 5 Hz).

### Exemple 13 :

Acide (4S,5R)-4-Phényloxazolidin-3-one-5-carboxylique

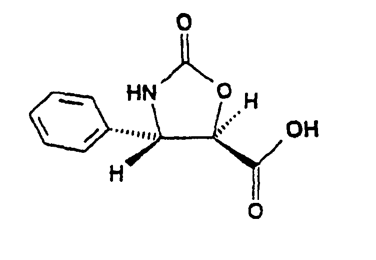

A une solution agitée à 0°C sous atmosphère inerte de 300 mg (0,867 mmol) de (4S,5R)-4-phényloxazolidin-2-one-5-carboxylate de ( 1S,2R,5S)-(+)-menthyle, 3 mL de méthanol, puis 0.5 mL d'eau dans 6,5 mL de pyridine, on additionne rapidement 10 mL d'une solution homogène de 360 mg (8,67 mmol) de NaOH, 3 mL de méthanol et 0,5 mL d'eau dans la pyridine. Après 20 min d'agitation à 0°C, le milieu réactionnel est dilué avec de l'eau (30 mL) et lavé avec du dichorométhane (30 mL). Après acidification à pH=1, la phase aqueuse résiduelle est extraite à l'acétate d'éthyle (3 x 20 mL), ct les phases organiques réunies sont séchées (MgSO₄), et concentrée sous pression réduite.

On obtient ainsi 86 mg d'acide (4S,5R)-4-phényloxazolidin-3-one-5-carboxylique sous la forme d'un sirop jaune (Rdₜ = 53%), et qui présente les caractéristiques suivantes:
- RMN ¹H 400MHz (DMSO-d₆) (δ ppm): 13,33 (1H, s large); 8,46 (1H, s): 7,38 (5H, m); 4,89 (1H, d, J = 5 Hz); 4,75 (1H, d, J= 5 Hz).

### II. Dérivés de la baccatine III

### Exemple 14 :

7-*O*-Triéthylsilyl-10-désacétylbaccatine III

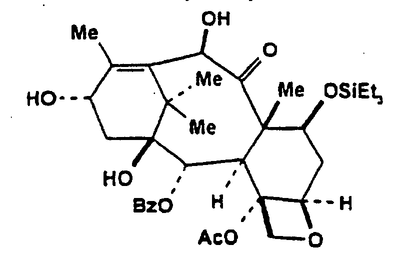

A une solution agitée à température ambiante et sous atmosphère inerte de 10 g (18,3 mmol) de 10-désacétylbaccatine III et de 8,17 g (54,9 mmol) de 4-pyrrolidinopyridine dans 500 mL de dichlorométhane anhydre, on ajoute 6,2 mL (36,6 mmol) de chlorure de triéthylsilyle en 10 min. Après 3 h de réaction à température ambiante, on ajoute 10 g de glace pilée et le mélange est laissé 10 min sous forte agitation. La phase organique résiduelle est lavée à l'eau (200 mL), séchée sur MgSO₄ et concentrée sous pression réduite.

Après traitement du produit brut obtenu avec le minimum d'acétate d'éthyle, on obtient 11,2 g de 7-*O*-triéthylsilyl-10-désacétylbaccatine III à l'état cristallisé (Rdt = 92,3 %).

Le produit ainsi obtenu présente les caractéristiques suivantes:
- RMN ¹H 400 MHz (CDCl₃) (δ ppm): 8,10 (2H, d, J = 7,4 Hz); 7,60 (1H, t, J = 7,5 Hz); 7,47 (2H, t, J = 7,6 Hz); 5,60 (1H, d, J= 7 Hz); 5,17 (1H, d, J = 1,9 Hz); 4,96 (1H, d, J = 8 Hz); 4,86 (1H, m); 4,41 (1H, dd, J = 10,6 et 6,6 Hz); 4,31 et 4,16 (2H, 2d, J = 8,4 Hz); 4,26 (1H, d, J = 1,9 Hz); 3,95 (1H, d, J = 6,9 Hz); 2,48 (1H, ddd, J = 1 4,5, 9,7 et 6,7 Hz); 2,29 (3H, s); 2,27 (2H, m): 2,08 (3H, s); 1,90 (1H, m); 1,73 (3H, s); 1,62 (1H, s); 1,08 (6H, s); 0,94 (9H, t, J = 8 hz); 0,56 (6H, m).

### Exemple 15 :

7-*O*-Triéthylgermanyl-10-désacétylbaccatine III

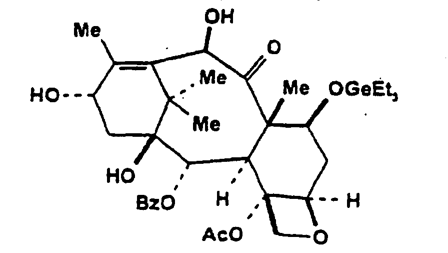

A une solution agitée à température ambiante ct sous atmosphère inerte de 100 mg (0,183 mmol) de 10-désacétylbaccatine III et de 41 mg (0,275 mmol) de 4-pyrrolidinopyridinc dans 4 mL de dichlorométhane anhydre, on ajoute 80 µL (0,476 mmol) de chlorure de triéthylgermanyle en 10 min, et le mélange est agité à 50°C pendant 13 h. Après refroidissement du milieu réactionnel, et dilution avec 15 mL de dichlorométhane, on ajoute 1 g de glace pilée et le mélange est laissé 10 min sous forte agitation. La. phase organique résiduelle est lavée avec une solution saturée d'hydrogénocarbonate de sodium (5 mL), une solution saturée de chlorure desodium (5 mL), séchée sur MgSO₄ ct concentrée sous pression réduite. Après chromatographie du produit brut sur gel de silice (15-40 µm)(éluant: cyclohexane-acétate d'éthyle, 25/75) on obtient 67 mg de 7-*O*-triéthylgermanyl-10-désacétylbaccatine III sous la forme d'un sirop incolore.

Le produit ainsi obtenu présente les caractéristiques suivantes:
- RMN ¹H 400 Mhz (CDCl₃)(δ ppm): 8,09 (2H, d, J = 7,1 Hz); 7,60 (1H, t, J = 7,4 Hz); 7,48 (2H, t, J = 7,6 Hz); 5,63 (1H, d, J = 7,1 Hz); 5,24 (1H, s); 4,99 (1H, d, J = 8 Hz); 4,78 (1H, t); 4,32 (1H, d, J = S,3); 4,28 (1H, m); 4,17 (2H, m); 3,97(1H, d, J = 7 Hz); 2,59 (1H, m); 2,30 (3H, s); 2,24 (1H, m); 2,10 (1H, m); 2,03 (3H, s); 1,82 (1H, m); 1,73 (3H, s); 1,11 (9H, m); 1,0 (6H, t, J = 7,7 Hz).

### Exemple 16 :

7-*O*-(2,2,2-Trichloro-*t*-butoxycarbonyl)-10-désacétylbaccatine III 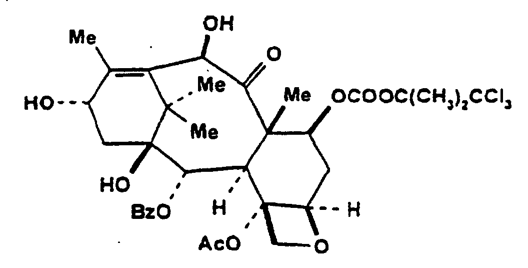 A une solution agitée à 40°C sous atmosphère inerte de 5g (9,19 mmol) de 10-désacétylbaccatine III ct 1,1 mL de pyridine anhydre dans 250 mL de dichlorométhane sec, on additionne 3,3 g (13,8 mmol) de chlorure de 2,2,2-trichloro-*t*-butoxycarbonyle en 2 h. Après 30 min de réaction supplémentaires, ct retour à température ambiante, la solution organique est lavée avec une solution aqueuse d'HCl 2% (30mL), lavée avec de l'eau osmosée (2 x 100 mL), séchée sur MgSO₄ et concentrée sous pression réduite (Rdt = 55%). Après chromatographie du produit brut sur gel de silice (15-40 µm) (éluant : cyclohexane-acétate d'éthyle, 60/40) on obtient la 7-*O*-(2,2,2-trichloro-*t*-butoxycarbonyl)-10-désacétylbaccatine III sous la forme d'une poudre blanche.

Le produit obtenu présente les caractéristiques suivantes:
- RMN ¹H 400 MHz (CDCl₃) (δ ppm): 8,10 (2H, d, J = 7 Hz); 7,62 (1H, t, J = 7,4 Hz); 7,49 (2H, t, J = 7,6 Hz); 5,65 (1H, d, J = 6,9 Hz); 5,44 (1H, dd, J = 10,8 et 7,3 Hz); 5,39 (1H, d); 4,98 (1H, d, J = 7,5 Hz); 4,89 (1H, m); 4,35 et 4,20 (2H, 2d, J = 8,4 Hz); 4,10 (1H, d, J = 7Hz); 4,01 (1H, d, J = 1,8 Hz); 2,64 (1H, m); 2,31 (3H, s); 2,29 (1H, m); 2,11 (3H, d); 2,05 (2H, m); 1,89 (3H, s); 1,09 (3H, s); 107 (3H, s).

### Exemple 17 :

a) 7-*O*-Triéthylsilylbaccatine III

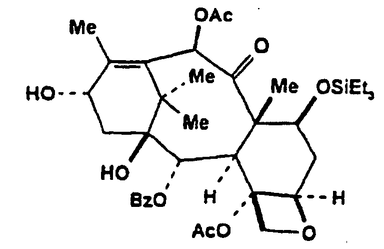

A une solution agitée à température ambiante sous atmosphère inerte de 1 g (1,5 mmol) de 7-*O*-triéthylsilyl-10-désacétylbaccatine III et de 1,25 mL (15 mmol) de pyridine dans 15 mL de dichlorométhanesec, on ajoute 0,54 mL (7,5 mmol) de chlorure d'acétyle en 10 min. Après 2 h de réaction à température ambiante et contrôle par C.C.M., on ajoute 1 g de glace pilée et le mélange est laissé sous forte agitation pendant 10 min. La phase organique résiduelle est lavée à l'eau (2 x 10 mL), séchée sur MgSO₄ et concentrée sous pression réduite. Après chromatographie sur gel de silice (15-40 µm) (éluant: cyclohexane-acétate d'éthyle, 60/40) on obtient 0,756 g de 7-*O*-triéthylsilylbaccatine III sous la forme d'une poudre blanche (Rdt = 70 %).

Le composé obtenu présente les caractéristiques suivantes:
- RMN ¹H 400 MHz (CDCl₃) (δ ppm): 8,11 (2H, d J = 7,1 Hz); 7,6 (1H, t, J = 7,4 Hz); 7,48 (2H, t, J = 7,7 Hz);6,46 (1H, s); 5,63 (1H, d, J = 7 Hz); 4,96 (1H, d, J = 8,1 Hz); 4,83 (1H, m); 4,49 (1H, dd, J = 10,4 et 6,7 Hz); 4,31 et 4,15 (2H, 2d, J = 8,3 Hz); 3,88 (1H, d, J = 7 Hz); 2,53 (1H, m); 2,29 (3H, s); 2,27 (2H, m); 2,19 (3H, d, J = 0,8 Hz); 2,18 (3H, s); 2,12 (1H, d); 1,88 (1H, m); 1,68 (3H, s); 1,65 (1H, s); 1,2 (3H, s); 1,04 (3H, s); 0,92 (9H, t); 0,59 (6H, m).

### Exemple 18 :

7-*O*-(2,2,2-Trichloro-*t*-butoxycarbonyl)baccatine III

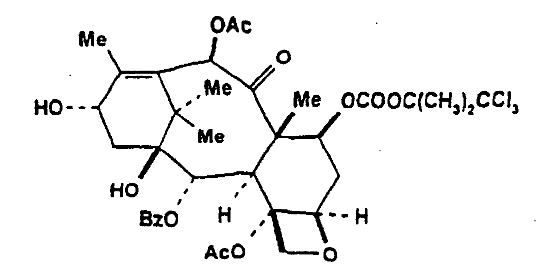

A une solution agitée à température ambiante sous atmosphère inerte de 260 mg de 7-*O*-(2,2,2-trichloro-*t*-butoxycarbonyl-10-désacétylbaccatine III et 127,5 mg (1,04 mmol) de 4-diméthylaminopyridine dans 2,5 mL de dichlorométhane sec, on ajoute 50 µL (0,695 mmol) de chlorure d'acétyle. Après 1 h de réaction à température ambiante, la phase organique est lavée avec une solution aqueuse d'HCl 2 % jusqu'à obtention d'un pH = 6, séchée sur Mg SO₄ et concentrée sous pression réduite. Après chromatographie du résidu obtenu sur gel de silice (15-40 µm) (éluant: cyclohexane-acétate d'éthyle, 6/4) on obtient 0,23 g de 7-O-(2,2,2-Trichloro-t-butoxycarbonyl)-baccatine III à l'état solide (Rdt = 83 %).

Le composé obtenu présente les caractéristiques suivantes:
- RMN ¹H 400 MHz (CDCl₃) (δ ppm): 8,11 (2H, d, J = 7,1 Hz); 7,62 (1H, t, J = 7,4 Hz); 7,49 (2H, t, J = 7,6 Hz); 6,39 (1H, s); 5,64 (1H, d, J = 6.9 Hz); 5,61 (1H, dd, J = 10,7 ct 7,2 Hz); 4,99 (1H, d, J = 8,2 Hz); 4,87 (1H, m); 4,33 et 4,16 (2H, 2d, J = 8,4 Hz); 4,02 (1H, d, J = 6,9 Hz); 2,64 (1H, ddd, J = 14,4, 9,5 et 7,2 Hz); 2,30 (3H, s) et (2H, m); 2,17 (3H, s); 2,13 (3H, d, J = 0,8 Hz); 2,04 (1H, m); 1,83 (3H, s); 1,63 (1H, s); 1,14 (3h, s); 1.09 (3H, s).

### Exemple 19 :

7-*O*-Phénoxyacétyl-10-désacétylbaccatine III

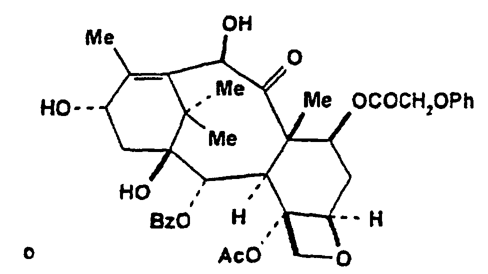

A une solution agitée à température ambiante et sous atmosphère inerte de 1,03 g (1,88 mmol) de 10-désacétylbaccatine III ct 0,6 mL (7,5 mmol) de pyridine anhydre dans 100 mL de dichlorométhane sec, on ajoute 1,05 mL (7,5 mmol) de chlorure de phénoxyacétyle en 10 min. Après 30 min de réaction à température ambiante et contrôle par C.C.M., la solution organique est lavée avec une solution aqueuse d'HCl à 2 % jusqu'à obtention d'un pH= 2, lavée avec de l'eau osmosée (2 x 50 mL), séchée sur MgSO₄ et concentrée sous pression réduite (Rdt= 70,5 %). Après chromatographie du produit brut sur gel de silice (15-40 µm) (éluant: cyclohexane-acétate d'éthyle, 60/40) on obtient la 7-*O*-phénoxyacétyl-10-désacétylbaccatine III sous la forme d'une poudre blanche.

Le produit obtenu présente les caractéristiques suivantes:
- RMN ¹H 400 MHz (CDCl₃) (δ ppm): 8,09 (2H, d, J = 7,3 Hz); 7,61 (1H, t, J = 7,4 Hz); 7,48 (2H, t, J = 7,6 Hz); 7,31 (2H, t, J = 7,7 Hz); 6,99 (3H, m); 6,42 (1H, s); 5,61 (1H, d,J = 7 Hz); 4,97 (1H, d, J = 7,8 Hz); 4,86 (3H, m); 4,44 (1H, dd, J = 10,6 et 6,8Hz); 4,30 et 4,15 (2H, 2d, J = 8,4 Hz); 3,86 (1H, d, J = 7 Hz); 2,56 (1H, m); 2,27 (3H, s); 2,27 (2H, m); 2,05 ((3H, s); 1,86 (1H, m); 1,68 (3H, s); 1,01 (3H, s); 0.98 (3H, s).

### Exemple 20 :

7,1 0-*O*-Di-(phénoxyacétyl)-10-désacétylbaccatineIII

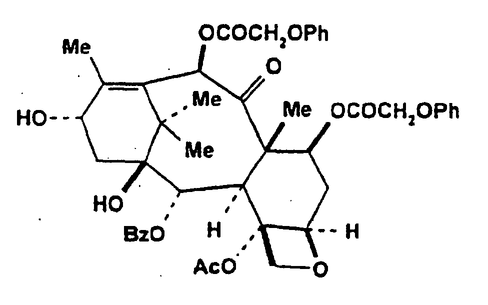

A une solution agitée à température ambiante et sous atmosphère inerte de 500 mg (0,92 mmol) de 10-désacétylbaccatine III et 0,6 mL (7,36 mmol) de pyridine anhydre dans 50 mL de dichlorométhane sec, on ajoute 0,5 mL (3,68 mmol) de chlorure de phénoxyacétyle en 10 min. Après 6 h de réaction à température ambiante et contrôle. par C.C.M., la solution est lavée avec une solution aqueuse d'HCl à 2 % jusqu'à obtention d'un pH = 2, lavée avec de l'eau osmosée (2 x 20 mL), séchée sur Mg SO₄ et concentrée sous pression réduite. Après chromatographie du produit brut sur gel de silice (15-40 µm) (éluant: cyclohexane-acétate d'éthyle, 6/4) on obtient 0,55 g de 7,10-*O*-bis-(phénoxyacétyl)-10-désacétylbaccatine III sous la forme d'une poudre blanche (Rdt = 74 %).

Le produit obtenu présente les caractéristiques suivantes:
- RMN ¹H 400 MHz (CDCl₃) (δ ppm): 8.09 (2H, d, J = 7,1 Hz); 7,61 (1H, t, J = 7,4 Hz); 7,48 (2H, t, J = 7,6 Hz); 7,29 (2H, t. J = 6.8 Hz); 7,22 (2H, t, J = 7,5 Hz); 6,96 (4H, m); 6,84 (2H, d, J = 7,9 Hz); 6,42 (1H, s); 5,69 (1H, dd, J = 10,5 et 7,1 Hz); 5.60 (1H, d, J = 6,9 Hz); 4,96 (1H, d, J = 8,2 Hz); 4,84 (1H, t, J = 7,4 Hz); 4,8 (2H, s); 4,65 et 4,41 (2H, 2d, J = 15,8 Hz); 4,32 et 4, 14 (2H, 2d, J = 8,4 Hz); 3,98 (1H, d, J = 6,8 Hz); 2,65 (1H, m); 2,28 (3H, s); 2,26 (2H, m); 2,09 (3H, s); 1,80 (3H, s) et (1H, m); 0,98 (6H, s).

### Exemple 21 :

7-*O*-Phénoxyacétylbaccatine III

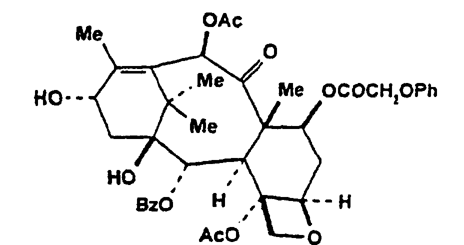

A une solution agitée à température ambiante et sous atmosphère inerte de 1,11 g (1,64 mmol) de 7-*O*-phénoxyacétyl-10-désacétylbaccatine III dans 40 mL de pyridine anhydre, on ajoute 0,233 mL (3,27 mmol) de chlorure d'acétyle en 10 min. Après 16 h de réaction à température ambiante et contrôle par C.C.M., le milieu réactionnel est dilué avec 50 mL d'eau osmosée, et la phase aqueuse est extraite à l'acétate d'éthyle (3 x 30 mL). Les phases aqueuses réunies sont lavées à l'eau (2 x 20 mL), séchées sur MgSO₄ et concentrées sous pression réduite (Rdt: 84,5 %). Après chromatographie sur gel de silice (15-40 µm) (éluant: cyclohexane-acétate d'éthyle, 60/40) on obtient la 7-*O*-phénoxyacétylbaccatine III à l'état cristallisé.

Le produit obtenu présente les caractéristiques suivantes:
- RMN ¹H 400 MHz (CDCl₃) (δ ppm): 8,10 (2H, d, J = 7,1 Hz); 7,61 (1H, t, J = 7,4 Hz); 7,48 (2H, t, J = 7,7 Hz); 7,27 (2H, t, J = S Hz): 6,95 (3H, m); 6,26 (1H; s); 5,71 (1H, dd, J = 10,4 et 7,2 Hz); 5,62 (1H, d, J = 6,9 Hz); 4,96 (1H, d, J = 8,3 Hz); 4,80 (1H, m); 4,81 et 4,53 (2H, 2d, J = 16 Hz); 4,32 et 4,14 (2H, 2d, J = 8,5 Hz); 4,0 (1H, d, J = 6,9 Hz); 2,64 (1H, m); 2,29 (2H, m); 2,28 (3H, s); 2,24 (1H, d, J = 5 Hz); 2,16 (3H, s); 2,09 (3H, d, J = 0,7 Hz); 1,81 (1H, m); 1,78 (3H, s); 1,13 (3H, s); 1,08 (3H, s).

### Exemple 22 :

7,10-*O*-(1,1,3,3-Tétraisopropyl-1,3-disiloxanediyl)-10-désacétyl baccatine III

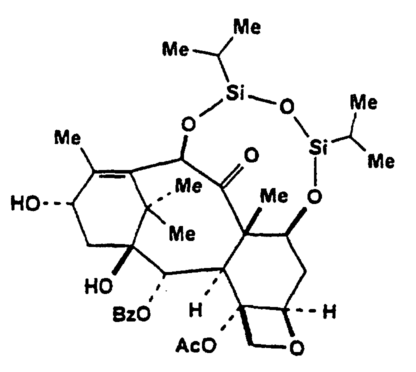

A une solution agitée à -40° C et sous atmosphère inerte de 500 mg (0,93 mmol) de 10-désacétylbaccatine III dans 20 mL de tétrahydrofurane anhydre, on ajoute 1,28 ml (2,05 mmol) de *n*-butyl lithium en solution 1,6 M dans l'hexane en 10 min. Après 5 min d'agitation, on ajoute 350 µL (1,12 mmol) de 1,3-dichloro-1,1,3,3-tétraisopropyldisyloxane et on laisse le milieu réactionnel revenir à température ambiante en 20 min. Après 1 h d'agitation à température ambiante, on additionne 225 mg (2,05 mmol) de 4-diméthylaminopyridine et le milieu réactionnel est laissé une 1 h supplémentaire sous agitation. Après addition de 20 mL d'une solution aqueuse saturée de chlorure de sodium, le milieu est extrait au dichlorométhane (3x 30 mL). Les phases organiques réunies sont lavées avec une solution aqueuse saturée de chlorure de sodium (20 ml), séchées sur MgSO₄ et concentrées sous pression réduite. Après purification par chromatographie sur gel de silice (15-40µm) (éluant: cyclohexane-acétate d'éthyle, 60/40) on obtient 480 mg de 7,10-*O*-(1,1,3,3-tétraisopropyl-1,3-disyloxanediyl)-10-désacétylbaccatineIII à l'état amorphe (Rdt = 65 %).

Le produit obtenu présente les caractéristiques suivantes:
- RMN ¹H 400 Mhz (CDCl₃) (δ ppm): 8,10 (2H, d, J = 7,2 Hz); 7,60 (1H, t, J = 7,4 Hz); 7,47 (2H, t, J = 7,6 Hz); 5,60 (1H, s); 5,59 (1H, d); 4,97 (1H, d, J = 7,9 Hz) 4,87 (1H, m); 4,68 (1H, dd, J = 10,4 et 6,9 Hz); 4,30 et 4,17 (2H, 2d, J = 8,5 Hz); 3,92 (1H, d, J = 7,1 Hz); 2,49 (1H, m); 2,28 (3H, s); 2,27 (1H, m); 2,04 (1H, m); 1,91 (1H, m); 1,67 (3H, s); 1,55 (1H, s); 1,32 à 0,85 (34 H, m).

### Exemple 23 :

13-*O*-[((4S,5R)-2,4-Diphényl-4,5-dihydroxazol-5-yl]-carbonyl]-7-*O*-triéthylsilyl-baccatine III

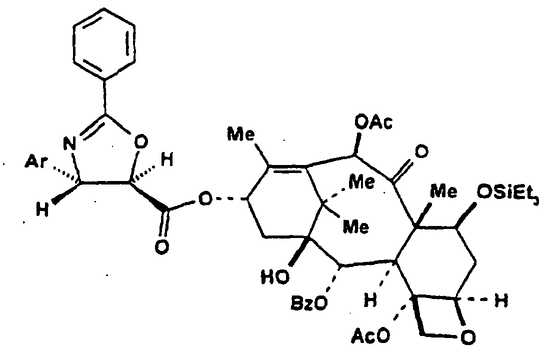

A une solution agitée à température ambiante et sous atmosphère inerte de 2,67 g (10 mmol) d'acide (4S,5R)-2,4-diphényl-4,5-dihydroxazol-5-carboxyliquedans 55 mL de toluène anhydre, on ajoute 2,06 g (10 mmol) de dicyclohexylcarbodiimidc. Après 5 min d'agitation, on ajoute 3,5 g (5 mmol) de 7-*O*-triéthylsilylbaccatine III et 0,61 g (5 mmol) de 4-diméthylamino-pyridine, ct le mélange réactionnel est porté à 70°C pendant 1 h. Après retour à température ambiante et élimination des insolubles par filtration, la phase organique est concentrée sous pression réduite. Après purification du produit brut par chromatographie sur gel de silice (15-25 µm) (éluant: cyclohexane-acétate d'éthyle, 90/10).

On obtient 4,62 g de 13-*O*-[[(4S.5R)-2,4-diphényl-4,5-dihydroxazol-5-yl]-carbonyl-7-*O*-triéthylsilylbaccatine III à l'état cristallisé (Rdt = 97 %).

Le composé ainsi obtenu présente les caractéristiques suivantes:
- RMN ¹H 400 MHz (CDCl₃) (δ ppm): 8,23 (2H, d, J = 7,2 Hz); 8,07 (2H, d, J = 7,3 Hz); 7,63 (1H, t, J = 7,4 Hz); 7,58 (1H, t, J = 7,4 Hz); 7,49 (4H, m); 7,38 (5H, m); 6,42 ((1H, s); 6,18 (1H, t, J = 8,2 Hz); 5,68 (1H, d, J = 7,1 Hz); 5,60 (1H, d, J = 6,5 Hz); 4,95 (2H, d); 4.50 (1H, dd, J = 10,5 et 6,7 Hz); 4,29 (1H, d, J = 8,4 Hz); 4,14 (1H, d, J = 8,4 Hz); 3,83 (1H, d, J = 7,1 Hz); 2,55 (1H,m); 2,37 (1H, dd, J = 15,3 et 9,3 Hz); 2,26 (1H, dd, J = 15,3 et 8,6 Hz); 2,16 (3H, s); 2,07 (3H, s); 1,99 (3H, s); 1,89 (1H, m); 1,72 (1H, s); 1,69 (3H, s); 1,23 (3H, s); 1,19 (3H, s); 0,92 (9H, t, J = S Hz); 0,57 (6H, m).

### Exemple 24 :

13-*O*-[[4S,5R)-2,4-Diphényl-4,5-dihydrooxazol-5-yl]-carbonyl]-7-*O*-phénoxyacétylbaccatine III

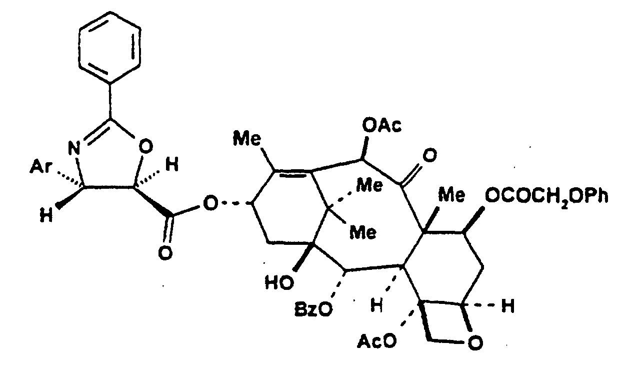

A une solution agitée à température ambiante et sous atmosphère inerte de 490 mg (1,83 mmol) d'acide (4S, 5R)-2,4-diphényl-4,5-dihydrooxazol-5-carboxylique dans 10 mL de toluène anhydre, on ajoute 380 mg (1,84 mmol) de dicyclohexylcarbodiimide. Après 5 min d'agitation, on ajoute 660 mg (0,92 mmol) de 7-*O*-phénoxyacétylbaccatine 111 et 112 mg (0.92 mmol) de 4-diméthylaminopyridine, et le mélange réactionnel est porté à 70°C pendant 2 h.
Après retour à température ambiante ct élimination des insolubles par filtration, la phase organique est concentrée sous pression réduite. Après purification du produit brut par chromatographie sur gel de silice (15-40 µm) (éluant: cyclohexane-acétate d'éthyle, 99/1), on obtient 800 mg de 13-*O*-[[4S,5R)-2,4-diphényl-4,5-dihydrooxazol-5-yl]-carbonyl-7-*O*-phénoxyacétylbaccatine III à l'état cristallisé (Rdt = 90 %).

Le composé ainsi obtenu présente les caractéristiques suivantes:
- RMN ¹H 400MHz (CDCl₃) (δ ppm): 8,18 (2H, d, J = 7 Hz); 8,07 (2H, d, J = 7,3 Hz); 7,63 (1H, t, J = 7,4 Hz): 7,59-7,32 (10H, m); 7,28 (2H, t, J = 7,5 Hz); 6,94 (3H, m); 6,23 (1H,s) et (1H, m); 5,70 (1H, dd, J = 10,4 et 7,1 Hz); 5,67 (1H, d, J = 7,3 Hz); 5,58 (1H, d, J = 7 Hz); 4,93 (2H, d); 4,79 et 4,53 (2H, 2d, J = 15,9 Hz); 4,30 et 4,13 (2H, 2d, J = 8,5 Hz); 3,97 (1H, d, J = 6,9 Hz); 2,67 (1H, m); 2,38 (1H, dd, J = 15,2 et 9,3 Hz); 2,26 (1H, dd, J = 15,2 et 8,4 Hz); 2,15 (3H, s); 2,02 (3H, s); 1,95 (3H, s) et (1H, m); 1,80 (3H, s); 1,74 (1H, s); 1,25 (3H, s); 1,17 (3H, s).

### Exemple 25 :

13-*O*-[[(4S,5R)-2,4-Diphényl-4,5-dihydrooxazol-5-yl]-carbonyl]-7-*O*-(2,2,2-trichloro-*t*-butoxycarbonyl)baccatine III

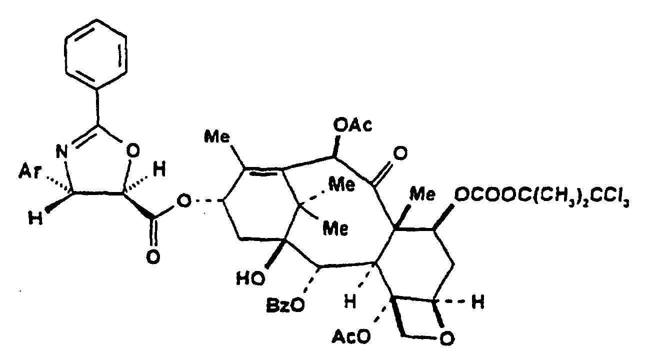

A une solution agitée à température ambiante et sous atmosphère inerte de 35 mg d'acide(4S,5R)-2,4-diphényl-4,5-dihydrooxazol-5-carboxylique dans 3 mL de toluène antydre, on ajoute 27 mg (0,13 mmol) de dicyclohexylcarbodiimide. Après 5 min d'agitation, on ajoute 51 mg (0,065 mmol) de 7-*O*-(2,2,2-trichloro-*t*-butoxycarbonyl)baccatine III et S mg (0.065 mmol) de 4-diméthylaminopyridine, et le mélange est porté à 70°C pendant 1h. Après retour à température ambiante et élimination des insolubles par filtration, la phase organique est concentrée sous pression réduite, et le résidu obtenu est purifié par chromatographie sur gel de silice (15-40 µm) (éluant: cyclohexane-acétate d'éthyle, 9/1).

On obtient ainsi 0,99 g du composé cité en titre sous la forme d'un solide blanc (Rdt = 67%), ct qui présente les caractéristiques suivantes:
- RNM ¹H 400 MHz (CDCl₃) (δ ppm): 8,18 (2H, d, J = 7,2 Hz); 8,07 (2H, d, J = 7,3 Hz); 7,65 (1H, t, J = 7,4 Hz); 7,59 (1H, t, J = 7,3 Hz); 7,52 (4H, m); 7,39 (5H, m); 6,35 (1H, s); 6,24 (1H, t, J = 8,4 Hz); 5,68 (1H, d, J = 7,1 Hz); 5,59 (1H, d, J = 7 Hz) ct (1H, dd); 4,95 (1H, d); 4,94 (1H, d, J = 7 Hz); 4,31 et 4,15 (2H,2d, J = 8,4 Hz); 3,97 (1H, d, J = 6,9 Hz); 2,64 (1H, m); 2,37 (1H, dd, J = 15,1 et 6 Hz); 2,27 (1H, dd, J = 15,2 et 8,5 Hz); 2,16 (3H, s); 2,01 (3H, s); 1,98 (3H, s); 1,83 (3H, s); 1,72 (1H, s); 1,25 (3H, s); 1,18 (3H, s).

### Exemple 26 :

13-*O*-[[4S,5R)-2,4-Diphényl-4,5-dihydrooxazol-5-yl]-carbonyl]-7,10-*O*-(1,1,3,3-tétraisopropyl-1,3-disiloxanediyl)-10-désacétylbaccatine III

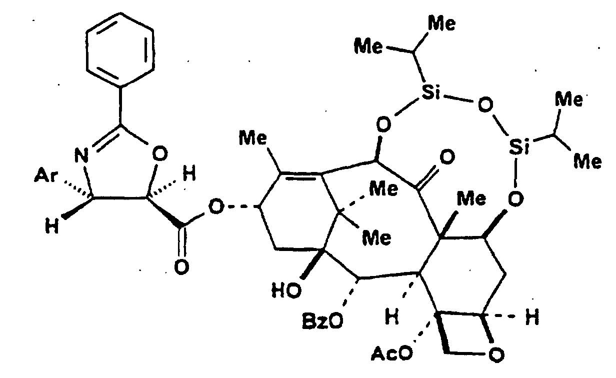

A une solution agitée à température ambiante, et sous atmosphère inerte de 4 mg (0,015 mmol) d'acide (4S,5R)-2,4-diphényl-4,5-dihydrooxazol-5-carboxylique dans 0,5 mL de toluène anhydre, on ajoute 7 mg (0,06 mmol) de dicyclohexylcarbodiimide. Après 5 min d'agitation, on ajoute une solution de 5 mg (0,0065 mmol) de 7,10-*O*-(1,1,3,3-tétraisopropyl-1,3-disiloxanediyl)-10-désacétylbaccatine III et de 1 mg (0,0078 mmol) de 4-diméthylaminopyridine dans 1 mL de toluène anhydre. Après 20 min d'agitation à température ambiante, le mélange est porté à 50° C pendant 20 min supplémentaires. Après retour à température ambiante, la phase organique est diluée avec 5 mL de dichlorométhane, lavée avec 2 mL d'une solution aqueuse saturée de chlorure de sodium, séchée sur MgSO₄ et concentrée sous pression réduite. Après purification du produit brut par chromatograhie sur gel de silice (15-25µm) (éluant: cyclohexane-acétate d'éthyle, 7/3) on obtient 6 mg du dérivé cité en titre (Rdt = 90 %) à l'état amorphe.

Le composé obtenu présente les caractéristiques suivantes:
- RMN ¹H 400 MHz (CDCl₃) (δ ppm): 8,21 (2H, d, J = 7,2 Hz); 8,07 (2H, d, J = 7,6 Hz); 7,63 (1H, t, J = 7,5 Hz); 7,59 (1H, t, J = 7,4 Hz); 7,50 (2H, t, J = 7.4 Hz); 7,39 (5H, m); 6,26 (1H, t); 5.64 (1H, d, J = 7 Hz); 5,59 (1H, d, J = 6,9 Hz); 5,54 (1H, s); 4,93 (1H, d, J = 6,8 Hz) et (1H, m); 4,68 (1H, dd); 4,28 ci 4,16 (2H, 2d, J = 8 Hz); 3,84 (1H, d, J = 7,3 Hz); 2,48 (1H, m); 2,35 et 2,25 (2H, 2dd); 2,02 (3H, s); 1,88 (3H, s) et (1H, m); 1,67 (3H, s); 1,63 (1H, s); 1,30 à 0,90 (34H, m).

### Exemple 27 :

13-*O*-[[(4S,5R)-3-*N*-Benzoyl-4-phényloxazolidin-3-one-5-yl]-carbonyl]-7-*O*-triéthylsilylbaccatine III

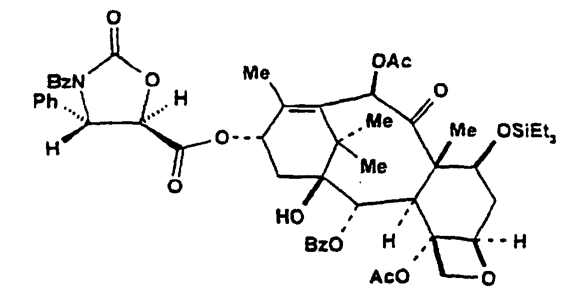

A une solution agitée sous atmosphère inerte à température ambiante de 40 mg (0,137 mmol) d'acide (4S,5R)-3-*N*-Benzoyl-4-phényloxazolidin-3-one-5-carboxylique dans 2 mL de toluène anhydre, on ajoute 28 mg (0,136 mmol) de dicyclohexylcarbodiimide. Après 5 min d'agitation, on ajoute 30 mg (0,043 mmol) de 7-*O*-triéthylsilylbaccatine III et 8 mg (0.066 mmol) de 4-diméthylaminopyridine, et le mélange réactionnel est porté à 60° C pendant 13 h. Après retour à température ambiante, le milieu réactionnel est dilué avec 10 mL de dichlorométhane, et la phase organique est lavée avec 5 mL d'une solution saturée de chlorure de sodium, séchée sur MgSO₄ et concentrée sous pression réduite. Après purification par chromatographie sur gel de silice (15-14µm) (éluant: cyclohexane-acétate d'éthyle 2/1) on obtient 13 mg du dérivé cité en titre à l'état amorphe (Rdt = 31 %).

Le composé obtenu présente les caractéristiques suivantes:
- RMN ¹H 400 MHz (CDCl₃) (δ ppm): 8,06 (2H, d, J = 7,3 Hz); 7,72 (2H, d, J = 7 Hz); 7,63 (1H, t, J = 7,4 Hz); 7,58 (1H, t, J = 7,4 Hz); 7,54 à 7,44 (8H, m); 7,40 (1H, t); 6,44 (1H, s); 6,33 (1H, t); 5,73 (1H, d, J = 5,7 Hz); 5,67 (1H, d, J = 5,7 Hz); 4,96 (1H, d, J = 5,8 Hz); 4,88 (1H, d, J = 8,3 Hz); 4,45 (1H, dd, J = 10,4 et 6,6 Hz); 4,27 et 4,12 (2H, 2d, J = 8,3 Hz); 3,80 (1H, d, J = 7 Hz); 2,50 (1H, m); 2,26 (2H, m); 2,19 (3H, s); 2,07 (3H, s); 1,98 (3H s); 1,85 (1H, m); 1,76 (1H, s); 1,67 (3H, s); 1,24 (3H, s); 1,23 (3H, s); 0,91 (9H, t, J = 7,9 Hz); 0,56 (6H, m).

### Exemple 28 :

13-*O*-[[(4S,5R)-4-phényloxazolidin-3-one-5-yl]-carbonyl]-7,10-*O*-di-(phénoxyacétyl)-10-désacétylbaccatineIII

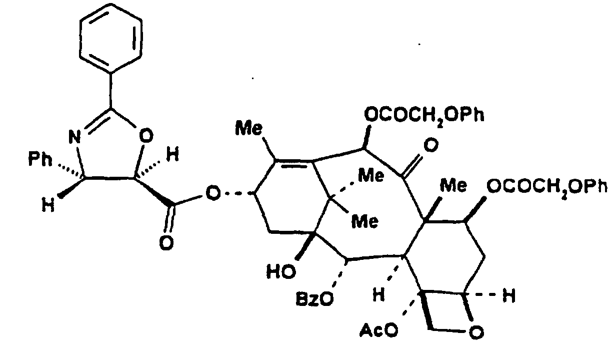

A une solution agitée à température ambiante et sous atmosphère inerte de 78 mg (0,293 mmol) d'acide (4S, 5R)-2,4-diphényl-4,5-dihydrooxazol-5-carboxylique dans 3 mL de toluène anhydre, on ajoute 65 mg (0,315 mmol) de dicyclohexylcarbodiimide. Après 5 min d'agitation, on ajoute une solution de 237 mg (0,293 mmol) de 7,10-*O*-bis-(phénoxyacétyl)-10-désacétylbaccatine III et 36 mg (0,295 mmol) de 4-diméthylaminopyridine dans 3 mL de toluène, et le mélange réactionnel est porté à 60°C pendant 1 h. Après retour à température ambiante et élimination des insolubles par filtration, la phase organique est concentrée sous pression réduite, et le produit brut obtenu est purifié par chromatographie sur gel de silice (15-40 µm) (éluant: cyclohexane-acétate d'éthyle, 1/1).

On obtient ainsi 280 mg du composé cité en titre à l'état amorphe (Rdt = 90 %), et qui présente les caractéristiques suivantes:
- RMN ¹H 400MHz (CDCl₃) (δ ppm): 8,18 (2H, d, J = 7 Hz); 8,06 (2H, d, J = 7,1 Hz); 7,64 (1H, t, J = 7,4 Hz); 7,58 (1H, t, J = 7,3 Hz); 7,51 (4H, m); 7,39 (5H, m); 7,25 (4H, m); 6,96 (4H, m); 6,85 (2H, d, J = 8 Hz); 6,33 (1H, s); 6,19 (1H, t, J = 9 Hz); 5,68 (1H, dd, J = 10,5 et 7,1 Hz); 5,65 (1H, d, J = 6,9 Hz); 5,59 (1H, d, J = 7 Hz); 4,93 (2H, d, J = 7,1 Hz); 4,79 (2H, s); 4,63 et 4,40 (2H, 2d, J = 15,9 Hz); 4,30 et 4,13 (2H, 2d, J = 8,4 Hz); 3,94 (1H, d, J = 6,9 Hz); 2,68 (1H, m); 2,37 (1H, dd, J = 15,3 ct 9,3 Hz); 2,24 (1H, dd, J = 15,3 et 8,7 Hz); 2,02 (3H, s); 1,95 (3H, s); 1,80 (3H, s) et (1H, m); 1,69 (1H, s); 1,12 (3H, s); 1,01 (3H, s).

### III. Hémisynthèse

### Exemple 29 :

### Préparation du paclitaxel

### a) A partir de la 13-O-[[(4S,5R)-2,4-diphényl-4,5-dihydrooxazol-5-yl]-carbonyl]-7-O-triéthylsilylbaccatine III

A une solution agitée à température ambiante et sous atmosphère inerte de 90 g (0,095 mol) de 13-*O*-[[(4S,5R)-2,4-diphényl-4,5-dihydrooxazol-5-yl)-carbonyl]-7-*O*-triéthylsilylbaccatine III dans un mélange de tétrahydro-furane (1,2 L) et de méthanol (1,2 L), on additionne 0,6 L (0,6 mol) d'une solution aqueuse d'HCI 1M, et le mélange réactionnel est agité à température ambiante pendant 4 h 30. Après addition de 3,5 L d'une solution aqueuse saturée d'hydrogénocarbonatede sodium, la solution est maintenue homogène par addition de 6 L de tétrahydrofurane ct 6 L d'eau, et le milieu réactionnel est agité 1 h 30 supplémentaire. Après addition de 15 L d'acétate d'éthyle et 15 L d'eau osmosée, la phase aqueuse résiduelle est extraite à l'acétate d'éthyle (15 L). La phase organique est séchée sur MgSO₄, concentrée sous pression réduite, et le produit brut ainsi obtenu est purifié par chromatographie sur gel de silice (15-40 µm) (éluant: cyclohexane-acétate d'éthyle, 1/1).

On isole ainsi 75 g de taxol à l'état cristallisé (Rdt = 95 %) dont les caractéristiques sont en tout point conformes aux données de la littérature.

### b) A partir de 13-O-[[(4S,5R)-2,4-diphényl-4,5-dihydrooxazol-5-yl]-carbonyl]-7-O-(2,2,2-trichloro-t-butoxycarbonyl)-baccatine III

A une solution agitée à température ambiante et sous atmosphère inerte de 15 mg (0,0148 mmol) de 13-*O*-[[(4S,5R)-2,4-diphényl-4,5-dihydrooxazol-5-yl]-carbonyl]-7-O-(2,2,2-trichloro-t-butoxycarbonyl)baccatine III dans un mélange de tétrahydrofurane (0,18 mL) et de méthanol (0,18 mL), on additionne 90 µL (0,09 mmol) d'une solution aqueuse d'HCl 1 M, et le mélange réactionnel est agité à température ambiante pendant S h. Après addition de 0,6 mL d'une solution aqueuse saturée d'hydrogénocarbonate de sodium, la solution est maintenue homogène par addition de 1 mL de tétrahydrofurane et 1 mL d'eau, et le milieu réactionnel est agité 1 h 30 supplémentaire. Après addition de 2,5 mL d'acétate d'éthyle et 2,5 mL d'eau osmosée, la phase aqueuse résiduelle est extraite à l'acétate d'éthyle (2,5 mL). Les phases organiques réunies sont séchées sur Mg SO₄, et concentrées sous pression réduite.

On obtient ainsi 14 mg de 7-O-(2,2,2-trichloro-t-butoxycarbonyl)-taxol à l'état brut (Rdt = 93 %) que l'on utilise sans autre purification dans l'étape suivante.

A une solution agitée à température ambiante de 13 mg (0,0128 mmol) de 7-O-(2,2,2-trichloro-t-butoxycarbonyl)-taxol dans 2 mL d'acétate d'éthyle, on ajoute 30 µL (0.525 mmol) d'acide acétique et 22,5 mg (0,344 mmol) de poudre de zinc. Après 2 h 30 d'agitation à température ambiante et contrôle par C.C.M., et après dilution du milieu réactionnel avec 3 mL d'acétate d'éthyle, la phase organique est lavée avec de l'eau osmoséc (1 mL), avec une solution aqueuse saturée d'hydrogénocarbonate de sodium (1 mL), à nouveau avec de l'eau, séchée sur Mg SO₄ et concentrée sous pression réduite.

Après chromatographie du produit brutsur gel de silice (15-40 µm) (éluant: cyclohexane-acétate d'éthyle, 6/4) on isole ainsi 9,5 mg de taxol à l'état cristallisé (Rdt = 89 %).

## Revendications

1. Procédé de préparation de précurseurs de chaîne latérale de taxanes dans lequel on transforme un dérivé cis-β-aryl-glycidate de formule générale I 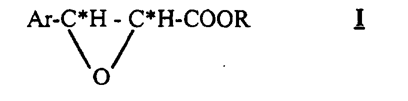 dans laquelle
Ar représente un radical aryle, et
R représente un radical hydrocarboné, de préférence un alkyle linéaire ou ramifié ou un cycloalkyle éventuellement substitué par un ou plusieurs groupes alkyles,
de manière à mettre en place régio- et stéréo-spécifiquement la β-Nalcoylamide et l'α-hydroxyle ou leurs précurseurs cycliques en une seule étape par une réaction de Ritter, qui consiste soit :
**a** en la synthèse directe d'une chaîne linéaire en faisant réagir un dérivé cis-β-aryl-glycidate de formule générale I défini ci-dessus, avec un nitrile de formule
R₂-CN
dans laquelle
R₂ représente un radical aryle,
en présence d'un acide protonique et d'eau, pour obtenir un dérivé de la β-aryl-isosérine de formule générale IIa, 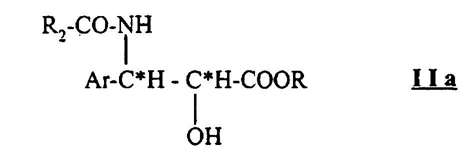 dans laquelle Ar, R et R₂ sont définis précédemment ; soit
**b** en la synthèse directe d'une chaîne cyclique en faisant réagir un dérivé cis-β-aryl-glycidate de formule générale I défini ci-dessus, avec un nitrile de formule
R'₂-CN
dans laquelle
R'₂ représente R₂ défini précédemment, ou un radical alkyle inférieur ou perhalogénoalkyle inférieur, tel que le trichlorométhyle,
en présence d'un acide de Lewis ou d'un acide protonique, en milieu anhydre, pour obtenir l'oxazoline de formule générale IIb 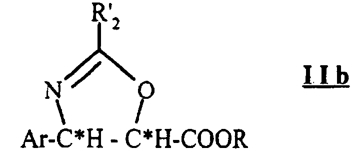 dans laquelle Ar, R et R'₂ sont définis précédemment.

2. Procédé selon la revendication 1, **caractérisé en ce que** R représente un énantiomère optiquement pur d'un radical hydrocarboné chiral de fort encombrement stérique, avantageusement un cycloalkyle substitué par un ou plusieurs groupes alkyle, en particulier un cyclohexyle.

3. Procédé selon la revendication 2, **caractérisé en ce que** R est l'un des énantiomères du radical menthyle, en particulier le (+)-menthyle.

4. Procédé selon l'une des revendications 1 à 3, **caractérisé en ce que** le dérivé cis-β-phényl-glycidate de formule générale I est de configuration (2R,3R) et les dérivés de formules générales IIa et IIb obtenus sont de configuration (2R,3S).

5. Procédé selon l'une des revendications 1 à 4, **caractérisé en ce que** Ar et R₂ représentent un phényle.

6. Procédé selon l'une des revendications 1 à 5, **caractérisé en ce que** l'acide protonique dans l'étape **a** est choisi parmi l'acide sulfurique, l'acide perchlorique ou l'acide tétrafluoroborique, l'acide de Lewis dans l'étape **b** est choisi parmi le complexe trifluorure de bore acide acétique, l'éthérate de trifluorure de bore, le pentachlorure d'antimoine, le tétrachlorure d'étain ou le tétrachlorure de titane, et l'acide protonique dans l'étape **b** est l'acide tétrafluoroborique.

7. Procédé selon l'une des revendications 1 à 6, **caractérisé en ce que** le dérivé de la β-aryl-isosérine de formule générale IIa est transformé par protection de l'hydroxy par un groupe protecteur (GP) approprié, pour obtenir un dérivé de formule générale II'a 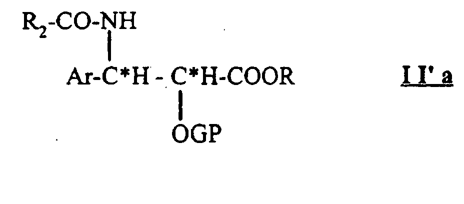 dans laquelle
Ar, R et R₂ sont définis comme à la revendication 1 et
GP représente un groupe protecteur de la fonction hydroxy, approprié pour la synthèse des taxanes, en particulier choisi parmi les radicaux alkoxyéther, aralkoxyéther, aryloxyéther ou haloalkoxycarbonyle, tels que par exemple les groupes méthoxyméthyle, 1-éthoxyéthyle, benzyloxy-méthyle, (β-triméthylsilyl-éthoxy)-méthyle, les radicaux tétrahydro-pyranyle, β-alkoxycarbonyle, les éthers β-halogénés, alkylsilylés, ou les radicaux alkoxyacétyle, aryloxyacétyle, haloacéthyle ou formyle.

8. Procédé selon l'une des redevendications 1 à 6, **caractérisé en ce que** le dérivé de la β-aryl-isosérine de formule générale IIa est transformé en de nouveaux intermédiaires cyclique, oxazolidinone de formule générale IIIa 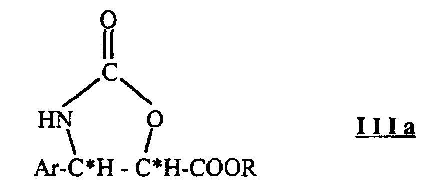 dans laquelle Ar et R sont définis comme à la revendication 1
en faisant réagir un dérivé de la β-aryl-isosérine de formule générale IIa selon l'une des revendications 1 à 5 avec un ester haloalkoxycarbonyle, en particulier le 2,2,2-trichloroéthoxycarbonyle (TrOC), puis par cyclisation en présence d'une base organique forte, telle que le diazabicyclo-undécène (DBU),
éventuellement transformée ensuite en l'amide correspondante de formule générale III'a 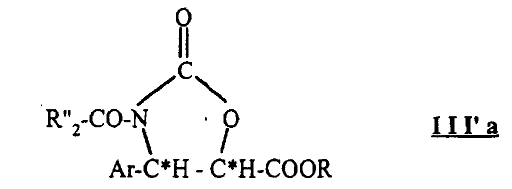 dans laquelle Ar et R sont définis comme à la revendication 1, et R"₂ représente R'₂ tel que défini à la revendication 1, un radical alkoxy, ou un radical alkyle, linéaire ou ramifié comprenant au moins une insaturation.

9. Procédé selon l'une des revendications 1 à 6, **caractérisé en ce que** l'on hydrolyse l'oxazoline de formule générale IIb en milieu acide, pour obtenir le dérivé de la β-aryl-isosérine de formule générale IIIb, 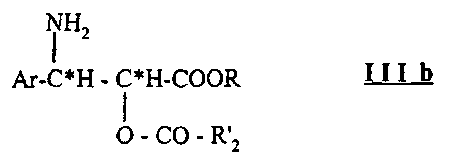 dans laquelle Ar, R et R'₂ sont définis comme à la revendication 1,
éventuellement transformé ensuite en l'amide correspondante de formule générale III'b 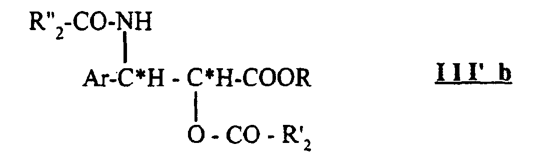 dans laquelle Ar, R, R'₂ et R"₂ sont définis comme à la revendication 1.

10. Procédé selon l'une des revendications 1 à 9, **caractérisé en ce que** le dérivé cis-β-aryl-glycidate de formule générale I 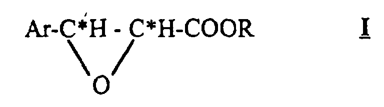 dans laquelle
Ar est défini comme à la revendication 1, et
R représente un énantiomère optiquement pur d'un radical hydro-carboné chiral de fort encombrement stérique,
est préparé en faisant réagir l'aldéhyde de formule
Ar-CHO
avec l'haloacétate de formule
X-CH₂-COOR
Ar, R étant définis comme à la revendication 1, et
X représentant un halogène, en particulier un chlore ou un brome.

11. Procédé selon l'une des revendications 1 à 10, **caractérisé en ce que** l'on obtient par saponification ménagée, les dérivés de formules IIa, II'a, IIb, IIIa, III'a, IIIb et III'b définis comme aux revendications 1, 7, 8 et 9 pour lesquels R représente un atome d'hydrogène.

12. Composés précurseurs de chaînes latérales de taxanes, **caractérisés en ce qu'**ils sont sélectionnés parmi les dérivés de formules générales I, IIa, IIb, II'a, IIIb, et III'b suivantes: 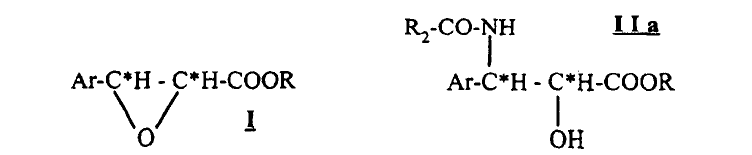 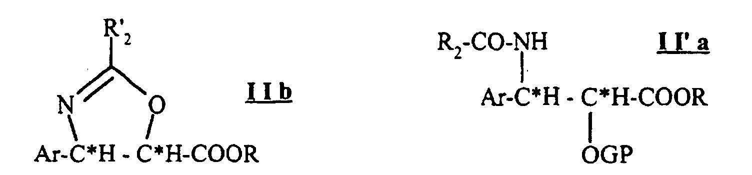 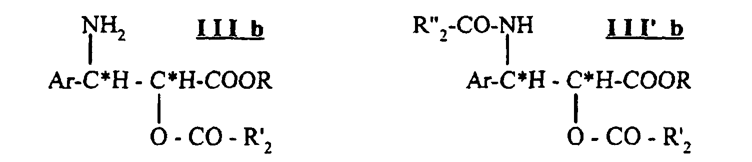 dans lesquelles
Ar, R₂, R'₂, R"₂ et GP sont définis dans l'une des revendications 1 à 3 et 5, et
R représente un énantiomère optiquement pur d'un radical hydro-carboné chiral de fort encombrement stérique.

13. Composés selon la revendication 12, **caractérisés en ce que** R est l'un des énantiomères du radical menthyle, en particulier le (+)-menthyle.

14. Composés selon l'une des revendications 12 ou 13, **caractérisés en ce que** le dérivé cis-β-phényl-glycidate de formule générale I est de configuration (2R,3R) et les dérivés de formules générales IIa, IIb, IIIb, et III'b sont de configuration (2R,3S).

15. Composés précurseurs de chaînes latérales de taxanes, **caractérisés en ce qu'**ils sont sélectionnés narmi les dérivés de formules générales IIIa et III'a suivantes: 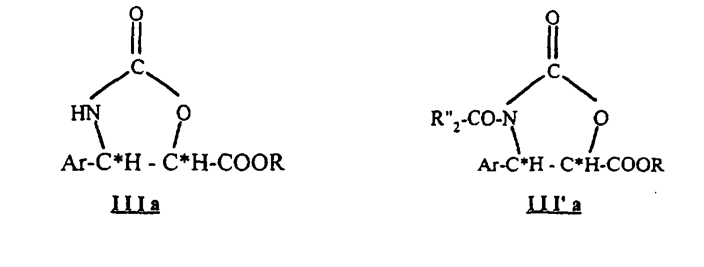 dans lesquelles
Ar, R et R"₂ sont définis comme aux revendications 1 et 8, ou R représente un atome d'hydrogène.

16. Composés selon la revendication 15, **caractérisés en ce qu'**ils sont de configuration (2R,3S).

17. Procédé de préparation de taxanes, de formule générale IV,
C-B **IV**
dans laquelle
B représente un radical de formule générale V 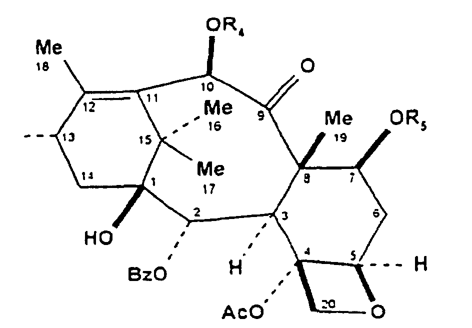 dans laquelle
Ac représente le radical acétyle,
Bz représente le radical benzyle,
Me représente le radical méthyle,
R₄ représente un radical acétyle ou un groupe protecteur de la fonction hydroxy GP1, et
R₅ représente un groupe protecteur de la fonction hydroxy GP2, et
C représente une chaîne latérale choisie parmi les radicaux de formules IIa, II'a, IIb, IIIa, III'a, IIIb et III'b suivantes : 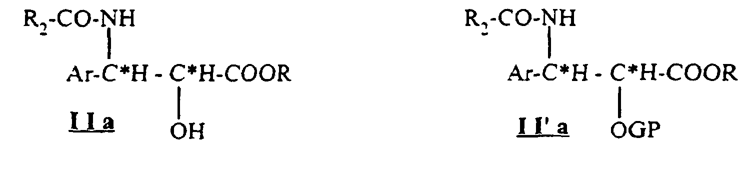 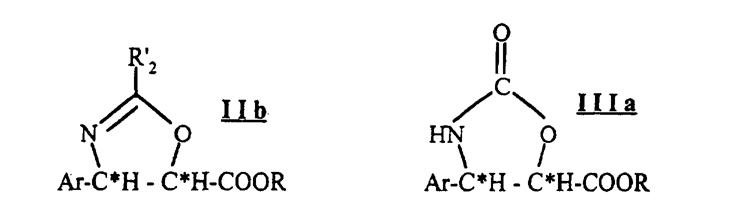 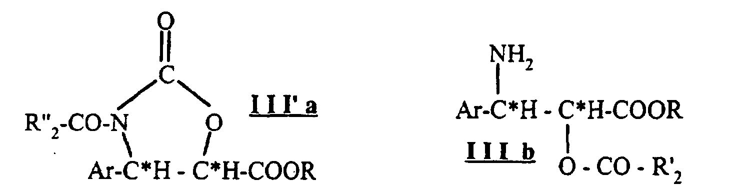 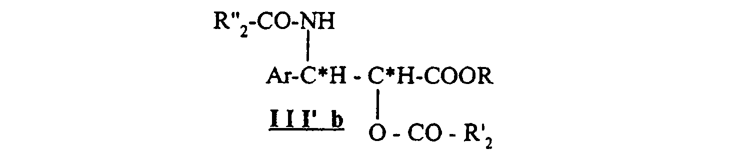
dans lesquelles Ar, R₂, R'₂, R"₂ et GP sont définis comme aux revendications 1, 7 et 8, par estérification d'un dérivé approprié de la baccatine III de formule générale V, porteur d'une fonction hydroxy en C-13, avec l'un des dérivés de formules IIa, II'a, IIb, IIIa, III'a, IIIb et III'b, pour lesquels R représente un atome d'hydrogène, obtenus par le procédé selon la revendication 11.

18. Procédé selon la revendication 17, **caractérisé en ce que** les groupements protecteurs GP1 et GP2 sont indépendamment l'un de l'autre des groupements usuels employés dans l'hémisynthèse des taxanes, tels que les trialkylsilyles ou le TROC, ou des radicaux haloalkoxycarbonyle encombrés, linéaires ou ramifiés comprenant au moins un atome d'halogène, des radicaux acyles dont le carbone en a de la fonction carbonyle porte au moins un atome d'oxygène, un radical trialkylgermanyle ou, GP1 et GP2 forment ensemble un radical divalent de formule
-SiR₇-O-SiR₈-
dans laquelle
R₇ et R₈, indépendamment l'un de l'autre représentent un radical alkyle encombré stériquement.

19. Procédé selon l'une des revendications 17 ou 18, **caractérisé en ce que** les radicaux acyles dont le carbone en a de la fonction carbonyle porte au moins un atome d'oxygène sont choisis parmi
- les radicaux alkoxy- ou aryloxyacétyle de formule
R₆-O-CH₂-CO-
dans laquelle R₆ représente un radical alkyle encombré stériquement, un radical cycloalkyle ou un radical aryle,
- ou les radicaux arvlidènedioxyacétyle de formule 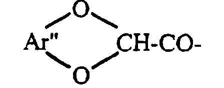 dans laquelle Ar" représente un radical arylidène.

20. Procédé selon la revendication 19, **caractérisé en ce que** :
le radical alkyle encombré stériquement est un radical alkyle linéaire ou ramifié en C₁-C₆ substitué par un ou plusieurs substituants encombrants choisi parmi les halogènes, les radicaux alkyle linéaires ou ramifiés en C₁-C₆, alkoxy linéaires ou ramifiés en C₁-C₆ ou cycloalkyle en C₃-C₆ ou aryle,
le radical cycloalkyle est un radical cycloalkyle en C₃-C₆ éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle, de préférence un radical cyclohexyle, substitué par un ou plusieurs radicaux alkyle linéaires ou ramifiés en C₁-C₆, par exemple le menthyle, son racémique ou ses énantiomères et leurs mélanges en toutes proportions,
le radical aryle, est un radical phényle, naphtyle, anthryle ou phénantryle, éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle, en particulier phényle, de préférence un radical phényle, éventuellement substitué par un ou deux substituants encombrants ci-dessus, en ortho- et ortho'- de la liaison éther, et
le radical arylidène est un radical phénylène, naphtyléne, anthrylène ou phénanthrylène, éventuellement substitué par un ou plusieurs substituants encombrants choisi parmi les halogènes, les radicaux alkyle linéaires ou ramifiés en C₁-C₆, alkoxy linéaires ou ramifiés en C₁-C₆ ou aryle, en particulier phényle.

21. Procédé selon l'une des revendications 17 ou 18, **caractérisé en ce que** R₄ représente un radical acétyle, et GP2 représente un radical trialkylsilyle, 2,2,2-trichloroéthoxycarbonyle, 2,2,2-tribromoéthoxy-carbonyle, 2,2,2,1-tétrachloroéthoxycarbonyle, 2,2,2-trichloro-*t*-butoxy-carbonyle, trichlorométhoxycarbonyle, phénoxyacétyle ou trialkyl-germanyle.

22. Procédé selon l'une des revendications 17 ou 18, **caractérisé en ce que** R₄ représente un groupe GP1, et GP1 et GP2 représentent un radical 2,2,2-trichloroéthoxycarbonyle ou phénoxyacétyle, ou forment ensemble un radical divalent de formule
-SiR₇-O-SiR₈-
dans laquelle R₇ et R₈ représentent chacun un radical isopropyle.

23. Procédé selon l'une des revendications 17 à 21, **caractérisé en ce que**
C représente un radical de formule IIa, avec Ar et R₂ représentent un phényle, et
R₄ représente un radical acétyle.

24. Procédé selon l'une des revendications 17 à 23, **caractérisé en ce que** l'on effectue ensuite la déprotection des hydroxy des dérivés de formule générale IV, et le cas échéant, simultanément ou séparément, l'ouverture du cycle oxazolinique des radicaux de formule IIb ou IIIa, pour obtenir un dérivé de taxane de formule générale VI 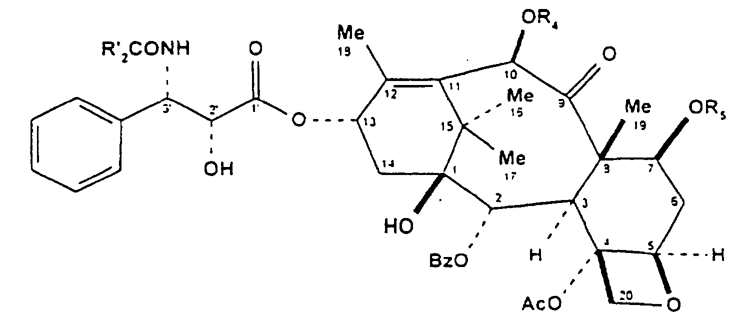 dans laquelle
Ac, Bz, Me et R'₂ sont définis dans l'une des revendications précédentes,
R₄ représente un atome d'hydrogène ou un radical acétyle, et
R₅ représente un atome d'hydrogène.

25. Dérivés de taxanes de formule générale IV
C-B **IV**
dans laquelle C et B sont définis dans l'une des revendications 17 à 23, à l'exception des dérivés pour lesquels C représente un radical de formule IIa, II'a, IIb, IIIb ou III'b, et GP1 et/ou GP2 sont indépendamment l'un de l'autre des groupements usuels employés dans l'hémisynthèse des taxanes, tels que les trialkylsilyles ou le TrOC.

26. Dérivés de la baccatine III, utiles pour l'hémisynthèse de taxanes, **caractérisés en ce qu'**ils sont choisis parmi les dérivés de formule générale V, 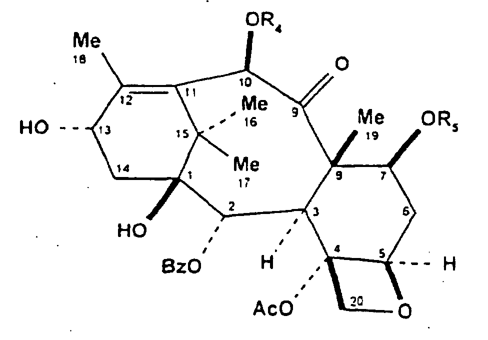 dans laquelle
Ac représente le radical acétyle,
Bz représente le radical benzoyle,
Me représente le radical méthyle,
R₄ représente un radical acétyle ou un groupe protecteur de la fonction hydroxy GP1,
R₅ représente un groupe protecteur de la fonction hydroxy GP2, et
GP1 et GP2 sont indépendamment l'un de l'autre des radicaux haloalkoxy-carbonyle encombrés, à l'exception du TrOC, des radicaux acyles dont le carbone en a de la fonction carbonyle porte au moins un atome d'oxygène, des radicaux trialkylgermanyle ou GP1 et GP2 forment ensemble un radical divalent de formule
-SiR₇-O-SiR₈-
dans laquelle
R₇ et R₈, indépendamment l'un de l'autre représentent un radical alkyle encombré stériquement.

## Claims

1. Process for the preparation of taxane side chain precursors in which a cis-β-arylglycidate derivative of general formula I 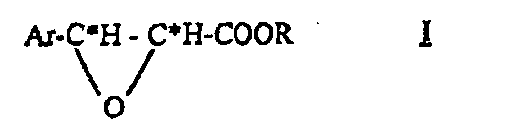 in which
Ar represents an aryl radical and
R represents a hydrocarbon radical, preferably a linear or branched alkyl or a cycloalkyl optionally substituted by one or more alkyl groups,
is converted, so as to regio- and stereospecifically introduce the β-Nalkylamide and the α-hydroxyl or their cyclic precursors in a single stage by a Ritter reaction, which consists either:
**a** of the direct synthesis of a linear chain by reacting a cis-β-arylglycidate derivative of general formula I defined above with a nitrile of formula
R₂-CN
in which
R₂ represents an aryl radical,
in the presence of a protonic acid and of water, in order to obtain a β-arylisoserine derivative of general formula IIa, 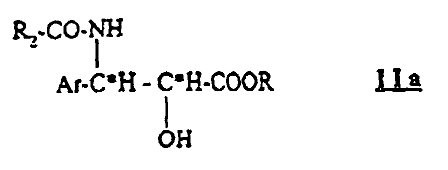 in which Ar, R and R₂ are defined above; or
**b** of the direct synthesis of a cyclic chain by reacting a cis-β-arylglycidate derivative of general formula I defined above with a nitrile of formula
R'₂-CN
in which
R'₂ represents R₂ defined above or a lower alkyl or lower perhaloalkyl radical, such as trichloromethyl,
in the presence of a Lewis acid or of a protonic acid, in anhydrous medium, in order to obtain the oxazoline of general formula IIb 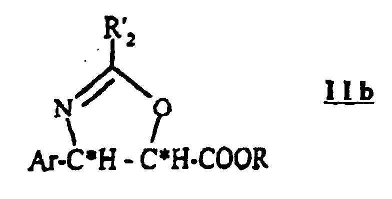 in which Ar, R and R'₂ are defined above.

2. Process according to Claim 1, **characterized in that** R represents an optically pure enantiomer of a highly sterically hindered chiral hydrocarbon radical, advantageously a cycloalkyl substituted by one or more alkyl groups, in particular a cyclohexyl.

3. Process according to Claim 2, **characterized in that** R is one of the enantiomers of the menthyl radical, in particular (+)-menthyl.

4. Process according to one of Claims 1 to 3, **characterized in that** the cis-β-phenylglycidate derivative of general formula I is of (2R,3R) configuration and the derivatives of general formulae IIa and IIb obtained are of (2R,3S) configuration.

5. Process according to one of Claims 1 to 4, **characterized in that** Ar and R₂ represent a phenyl.

6. Process according to one of Claims 1 to 5, **characterized in that** the protonic acid in the stage **a** is chosen from sulphuric acid, perchloric acid or tetrafluoroboric acid, the Lewis acid in the stage **b** is chosen from the boron trifluoride acetic acid complex, boron trifluoride etherate, antimony pentachloride, tin tetrachloride or titanium tetrachloride and the protonic acid in the stage **b** is tetrafluoroboric acid.

7. Process according to one of Claims 1 to 6, **characterized in that** the β-arylisoserine derivative of general formula IIa is converted by protection of the hydroxyl by an appropriate protective group (GP), in order to obtain a derivative of general formula II'a 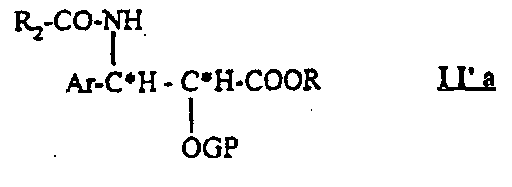 in which
Ar, R and R₂ are defined as in Claim 1 and
GP represents a protective group for the hydroxyl functional group which is appropriate for the synthesis of taxanes, in particular chosen from alkoxy ether, aralkoxy ether, aryloxy ether or haloalkoxycarbonyl radicals, such as, for example, methoxymethyl, 1-ethoxyethyl, benzyloxymethyl or (β-trimethylsilyl-ethoxy)methyl groups, tetrahydropyranyl or β-alkoxycarbonyl radicals, β-halogenated or alkylsilyl ethers, or alkoxyacetyl, aryloxyacetyl, haloacetyl or formyl radicals.

8. Process according to one of Claims 1 to 6, **characterized in that** the β-arylisoserine derivative of general formula IIa is converted into novel oxazolidinone cyclic intermediates of general formula IIIa 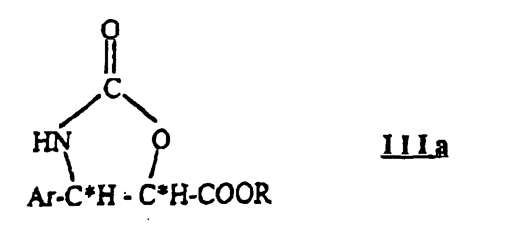 in which Ar and R are defined as in Claim 1
by reacting a β-arylisoserine derivative of general formula IIa according to one of Claims 1 to 5 with a haloalkoxycarbonyl ester, in particular 2,2,2-trichloroethoxycarbonyl (TrOC), and then by cyclization in the presence of a strong organic base, such as diazabicycloundecene (DBU),
optionally converted subsequently into the corresponding amide of general formula III'a 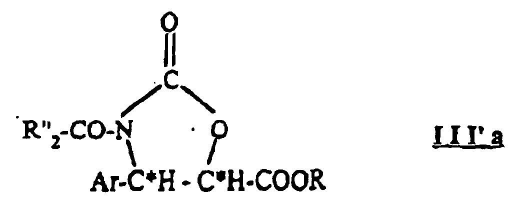 in which Ar and R are defined as in Claim 1 and R"₂ represents R'₂ as defined in Claim 1, an alkoxy radical or a linear or branched alkyl radical comprising at least one unsaturation.

9. Process according to one of Claims 1 to 6, **characterized in that** the oxazoline of general formula IIb is hydrolysed in acidic medium in order to obtain the β-arylisoserine derivative of general formula IIIb, 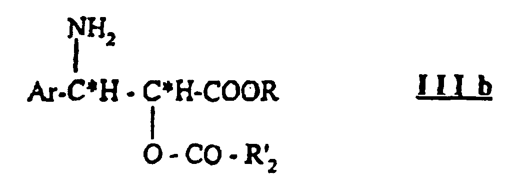 in which Ar, R and R'₂ are defined as in Claim 1,
optionally converted subsequently into the corresponding amide of general formula III'b 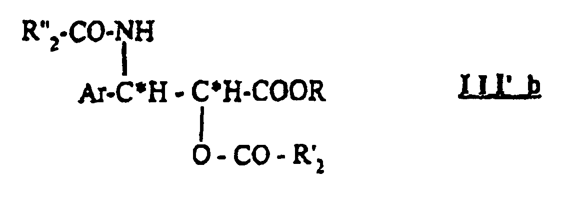 in which Ar, R, R'₂ and R"₂ are defined as in Claim 1.

10. Process according to one of Claims 1 to 9, **characterized in that** the cis-β-arylglycidate derivative of general formula I 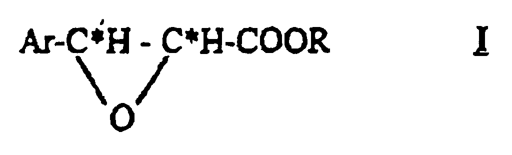 in which
Ar is defined as in Claim 1 and
R represents an optically pure enantiomer of a highly sterically hindered chiral hydrocarbon radical, is prepared by reacting the aldehyde of formula
Ar-CHO
with the haloacetate of formula
X-CH₂-COOR
Ar and R being defined as in Claim 1 and
X representing a halogen, in particular a chlorine or a bromine.

11. Process according to one of Claims 1 to 10, **characterized in that** the derivatives of formulae IIa, II'a, IIb, IIIa, III'a, IIIb and III'b defined as in Claims 1, 7, 8 and 9 in which R represents a hydrogen atom are obtained by controlled saponification.

12. Precursor compounds of taxane side chains, **characterized in that** they are selected from the derivatives of following general formulae I, IIa, IIb, II'a, IIIb and III'b; 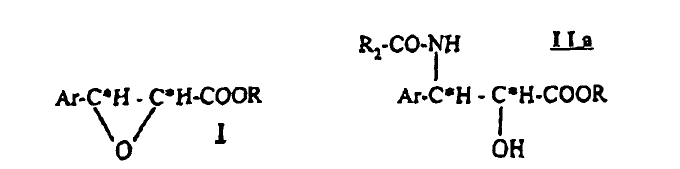 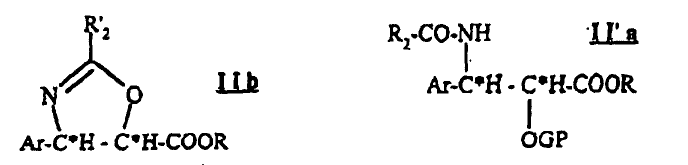 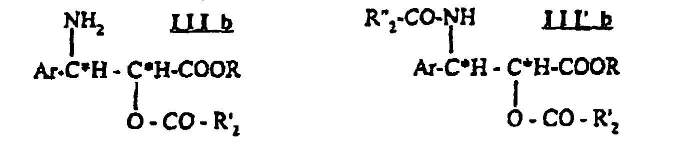 in which
Ar, R₂, R'₂, R"₂ and GP are defined in one of Claims 1 to 3 and 5, and
R represents an optically pure enantiomer of a highly sterically hindered chiral hydrocarbon radical.

13. Compounds according to Claim 12, **characterized in that** R is one of the enantiomers of the menthyl radical, in particular (+)-menthyl.

14. Compounds according to either of Claims 12 and 13, **characterized in that** the cis-β-phenylglycidate derivative of general formula I is of (2R,3R) configuration and the derivatives of general formulae IIa, IIb, IIIb and III'b are of (2R,3S) configuration.

15. Precursor compounds of taxane side chains, **characterized in that** they are selected from the derivatives of following general formulae IIIa and III'a: 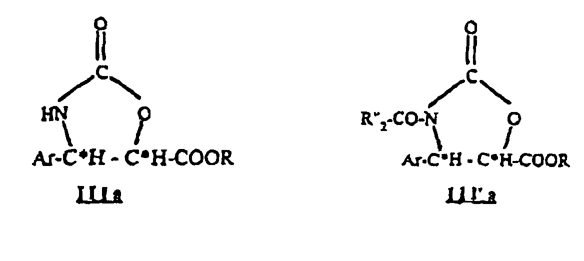 in which
Ar, R and R"₂ are defined as in Claims 1 and 8 or R represents a hydrogen atom.

16. Compounds according to Claim 15, **characterized in that** they are of (2R,3S) configuration.

17. Process for the preparation of taxanes of general formula IV,
C-B **IV**
in which
B represents a radical of general formula V 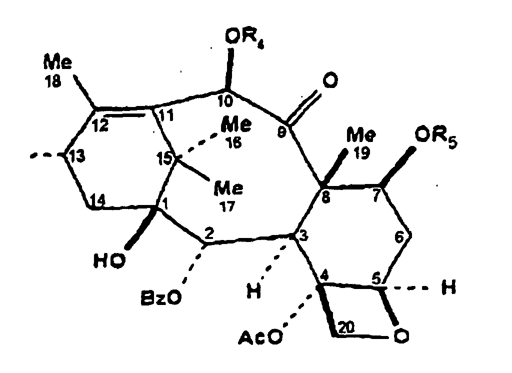 in which
Ac represents the acetyl radical,
Bz represents the benzyl radical,
Me represents the methyl radical,
R₄ represents an acetyl radical or a protective group for the hydroxyl functional group GP1, and
R₅ represents a protective group for the hydroxyl functional group GP2, and
C represents a side chain chosen from the radicals of following formulae IIa, II'a, IIb, IIIa, III'a, IIIb and III'b: 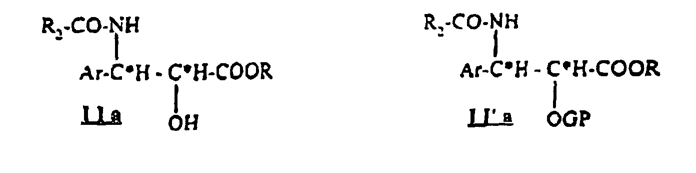 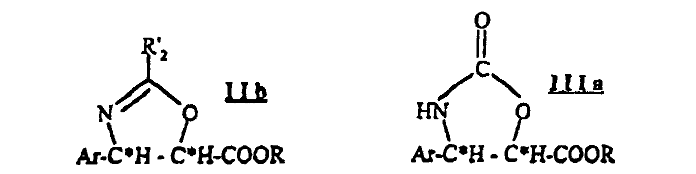 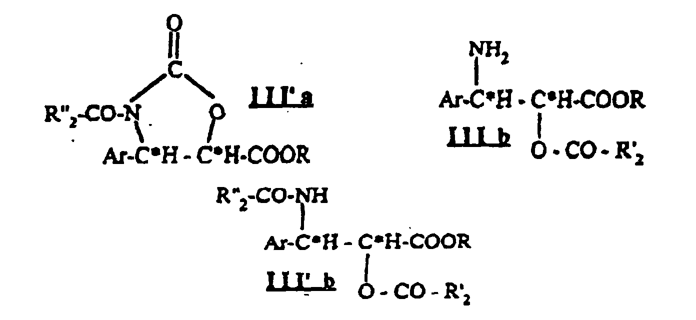
in which Ar, R₂, R'₂, R"₂ and GP are defined as in Claims 1, 7 and 8,
by esterification of an appropriate baccatin III derivative of general formula V, carrying a C-13 hydroxyl functional group, with one of the derivatives of formulae IIa, II'a, IIb, IIIa, III'a, IIIb and III'b, for which R represents a hydrogen atom, obtained by the process according to Claim 11.

18. Process according to Claim 17, **characterized in that** the GP1 and GP2 protective groups are, independently of one another, conventional groups employed in the hemisynthesis of taxanes, such as trialkylsilyls or TROC, or linear or branched bulky haloalkoxycarbonyl radicals comprising at least one halogen atom, acyl radicals in which the carbon α to the carbonyl functional group carries at least one oxygen atom, or a trialkylgermanyl radical or GP1 and GP2 together form a divalent radical of formula
-SiR₇-O-SiR₈-
in which
R₇ and R₈, independently of one another, represent a sterically hindered alkyl radical.

19. Process according to either of Claims 17 and 18, **characterized in that** the acyl radicals in which the carbon α to the carbonyl functional group carries at least one oxygen atom are chosen from
- alkoxy- or aryloxyacetyl radicals of formula
R₆-O-CH₂-CO-
in which R₆ represents a sterically hindered alkyl radical, a cycloalkyl radical or an aryl radical,
- or arylidenedioxyacetyl radicals of formula 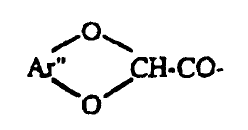 in which Ar" represents an arylidene radical.

20. Process according to Claim 19, **characterized in that**:
the sterically hindered alkyl radical is a linear or branched C₁-C₆ alkyl radical substituted by one or more bulky substituents chosen from halogens or linear or branched C₁-C₆ alkyl, linear or branched C₁-C₆ alkoxy or C₃-C₆ cycloalkyl or aryl radicals,
the cycloalkyl radical is a C₃-C₆ cycloalkyl radical optionally substituted by one or more bulky substituents chosen from halogens or linear or branched C₁-C₆ alkyl, linear or branched C₁-C₆ alkoxy or aryl radicals, preferably a cyclohexyl radical substituted by one or more linear or branched C₁-C₆ alkyl radicals, for example menthyl, its racemate or its enantiomers and their mixtures in all proportions,
the aryl radical is a phenyl, naphthyl, anthryl or phenanthryl radical optionally substituted by one or more bulky substituents chosen from halogens or linear or branched C₁-C₆ alkyl, linear or branched C₁-C₆ alkoxy or aryl radicals, in particular the phenyl radical, preferably a phenyl radical optionally substituted by one or two above bulky substituents ortho- and ortho'- to the ether bond, and
the arylidene radical is a phenylene, naphthylene, anthrylene or phenanthrylene radical optionally substituted by one or more bulky substituents chosen from halogens or linear or branched C₁-C₆ alkyl, linear or branched C₁-C₆ alkoxy or aryl radicals, in particular the phenyl radical.

21. Process according to either of Claims 17 and 18, **characterized in that** R₄ represents an acetyl radical and GP2 represents a trialkylsilyl, 2,2,2-trichloroethoxycarbonyl, 2,2,2-tribromoethoxycarbonyl, 2,2,2,1-tetrachloroethoxycarbonyl, 2,2,2-trichloro-t-butoxycarbonyl, trichloromethoxycarbonyl, phenoxyacetyl or trialkylgermanyl radical.

22. Process according to either of Claims 17 and 18, **characterized in that** R₄ represents a GP1 group and GP1 and GP2 represent a 2,2,2-trichloroethoxycarbonyl or phenoxyacetyl radical or together form a divalent radical of formula
-SiR₇-O-SiR₈-
in which R₇ and R₈ each represent an isopropyl radical.

23. Process according to one of Claims 17 to 21, **characterized in that**
C represents a radical of formula IIa with Ar and R₂ representing a phenyl and
R₄ represents an acetyl radical.

24. Process according to one of Claims 17 to 23, **characterized in that**, subsequently, the hydroxyls of the derivatives of general formula IV are deprotected and, if appropriate, simultaneously or separately, the oxazoline ring of the radicals of formula IIb or IIIa is opened, in order to produce a taxane derivative of general formula VI 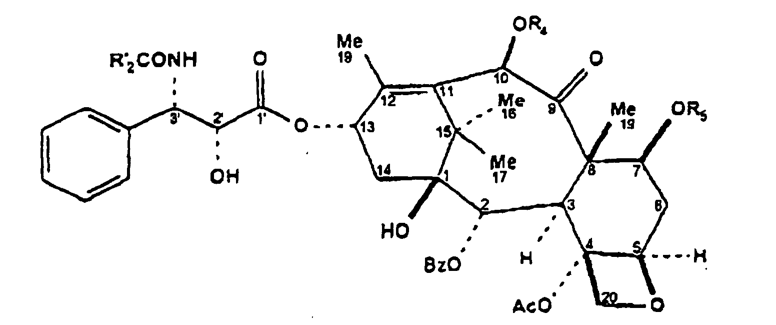 in which
Ac, Bz, Me and R'₂ are defined in one of the preceding claims,
R₄ represents a hydrogen atom or an acetyl radical and
R₅ represents a hydrogen atom.

25. Taxane derivatives of general formula IV
C-B **IV**
in which C and B are defined in one of Claims 17 to 23, with the exception of the derivatives in which C represents a radical of formula IIa, II'a, IIb, IIIb or III'b, and GP1 and/or GP2 are, independently of one another, conventional groups employed in the hemisynthesis of taxanes, such as trialkylsilyls or TrOC.

26. Baccatin III derivatives which are of use in the hemisynthesis of taxanes, **characterized in that** they are chosen from the derivatives of general formula V, 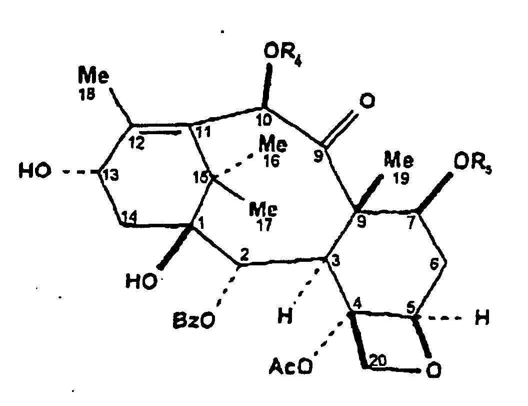 in which
Ac represents the acetyl radical,
Bz represents the benzoyl radical,
Me represents the methyl radical,
R₄ represents an acetyl radical or a protective group for the hydroxyl functional group GP1,
R₅ represents a protective group for the hydroxyl functional group GP2, and
GP1 and GP2 are, independently of one another, bulky haloalkoxycarbonyl radicals, with the exception of TrOC, acyl radicals in which the carbon α to the carbonyl functional group carries at least one oxygen atom, or trialkylgermanyl radicals or GP1 and GP2 together form a divalent radical of formula
-SiR₇-O-SiR₈-
in which
R₇ and R₈, independently of one another, represent a sterically hindered alkyl radical.

## Patentansprüche

1. Verfahren zur Herstellung von Vorstufen der Seitenkette von Taxanen, bei dem man ein cis-β-Aryl-glycidatderivat der allgemeinen Formel I 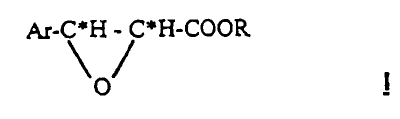 in der
Ar einen Arylrest darstellt und
R einen Kohlenwasserstoffrest darstellt, vorzugsweise einen linearen oder verzweigten Alkylrest oder einen Cycloalkylrest, der gegebenenfalls mit einer oder mehreren Alkylgruppen substituiert ist,
umwandelt, um die β-N-Alkylamidgruppe und die α-Hydroxygruppe oder ihre cyclischen Vorstufen regio- und stereospezifisch in einem einzigen Schritt durch eine Ritter-Reaktion einzuführen, die entweder besteht:
**a** aus der direkten Synthese einer linearen Kette, indem man ein cis-β-Aryl-glycidatderivat der oben definierten allgemeinen Formel I mit einem Nitril der Formel
R₂-CN
in der
R₂ einen Arylrest darstellt,
in Gegenwart einer Protonensäure und Wasser umsetzt, um ein β-Aryl-isoserinderivat der allgemeinen Formel IIa, 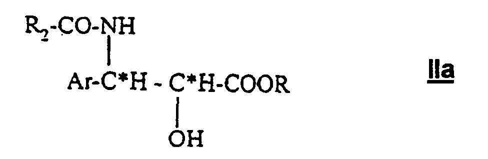 in der Ar, R und R₂ wie zuvor definiert sind, zu erhalten; oder
**b** aus der direkten Synthese einer cyclischen Kette, indem man ein cis-β-Aryl-glycidatderivat der oben definierten allgemeinen Formel I mit einem Nitril der Formel
R'₂-CN
in der
R'₂ den zuvor definierten Rest R₂ oder einen niedrigen Alkyl- oder niedrigen Perhalogenalkylrest, wie Trichlormethyl, darstellt,
in Gegenwart einer Lewis-Säure oder einer Protonensäure in wasserfreiem Medium umsetzt, um das Oxazolin der allgemeinen Formel IIb 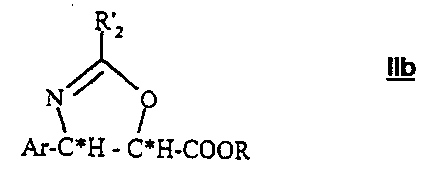 in der Ar, R und R'₂ wie zuvor definiert sind, zu erhalten.

2. Verfahren gemäß Anspruch 1, **dadurch gekennzeichnet, dass** R ein optisch reines Enantiomer eines chiralen Kohlenwasserstoffrests mit großem sterischen Raumbedarf, vorteilhafterweise einen mit einer oder mehreren Alkylgruppen substituierten Cycloalkylrest, insbesondere einen Cyclohexylrest, darstellt.

3. Verfahren gemäß Anspruch 2, **dadurch gekennzeichnet, dass** R eines der Enantiomere des Menthylrests, insbesondere (+)-Menthyl, ist.

4. Verfahren gemäß einem der Ansprüche 1 bis 3, **dadurch gekennzeichnet, dass** das cis-β-Phenyl-glycidatderivat der allgemeinen Formel I die (2R,3R)-Konfiguration hat und die erhaltenen Derivate der allgemeinen Formeln lla und IIb die (2R,3S)-Konfiguration haben.

5. Verfahren gemäß einem der Ansprüche 1 bis 4, **dadurch gekennzeichnet, dass** Ar und R₂ einen Phenylrest darstellen.

6. Verfahren gemäß einem der Ansprüche 1 bis 5, **dadurch gekennzeichnet, dass** die Protonensäure in der Stufe **a** unter Schwefelsäure, Perchlorsäure oder Tetrafluorborsäure ausgewählt ist, die Lewis-Säure in der Stufe **b** unter dem Bortrifluorid-Essigsäure-Komplex, Bortrifluorid-etherat, Antimonpentachlorid, Zinntetrachlorid oder Titantetrachlorid ausgewählt ist und die Protonensäure in der Stufe **b** Tetrafluorborsäure ist.

7. Verfahren gemäß einem der Ansprüche 1 bis 6, **dadurch gekennzeichnet, dass** das β-Aryl-isoserinderivat der allgemeinen Formel IIa durch Schützen der Hydroxyfunktion durch eine geeignete Schutzgruppe (GP) umgewandelt wird, um ein Derivat der allgemeinen Formel II'a 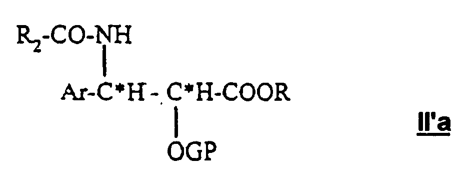 zu erhalten, in der
Ar, R und R₂ wie in Anspruch 1 definiert sind und
GP eine Schutzgruppe für die Hydroxyfunktion darstellt, die für die Synthese der Taxane geeignet und insbesondere ausgewählt ist unter den Resten Alkoxyether, Aralkoxyether, Aryloxyether oder Halogenalkoxycarbonyl, wie beispielsweise die Gruppen Methoxymethyl, 1-Ethoxyethyl, Benzyloxy-methyl, (β-Trimethylsilyl-ethoxy)-methyl, den Resten Tetrahydro-pyranyl, β-Alkoxycarbonyl, den β-Halogenalkylsilylethem oder den Resten Alkoxyacetyl, Aryloxyacetyl, Halogenacetyl oder Formyl.

8. Verfahren gemäß einem der Ansprüche 1 bis 6, **dadurch gekennzeichnet, dass** das β-Aryl-isoserinderivat der allgemeinen Formel IIa in neue cyclische Oxazolidinonzwischenstufen der allgemeinen Formel IIIa 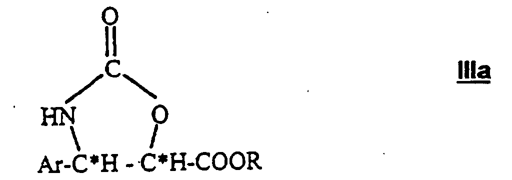 in der Ar und R wie in Anspruch 1 definiert sind,
umgewandelt wird, indem man ein β-Aryl-isoserinderivat der allgemeinen Formel IIa gemäß einem der Ansprüche 1 bis 5 mit einem Halogenalkoxycarbonylester, insbesondere 2,2,2-Trichlorethoxycarbonyl (TrOC), umsetzt, dann in Gegenwart einer starken organischen Base, wie Diazabicyclo-undecen (DBU), cyclisiert, wonach gegebenenfalls eine Umwandlung in das entsprechende Amid der allgemeinen Formel III'a 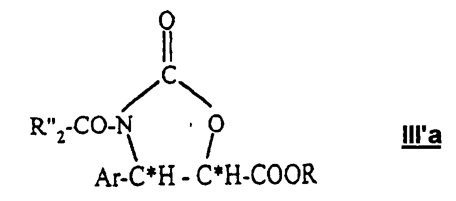 erfolgt, in der Ar und R wie in Anspruch 1 definiert sind und R"₂ R'₂, wie in Anspruch 1 definiert, einen Alkoxyrest oder einen Alkylrest darstellt, der linear oder verzweigt ist und wenigstens eine ungesättigte Bindung umfasst.

9. Verfahren gemäß einem der Ansprüche 1 bis 6, **dadurch gekennzeichnet, dass** man das Oxazolin der allgemeinen Formel IIb in saurem Medium hydrolysiert, um das β-Aryl-isoserinderivat der allgemeinen Formel IIIb 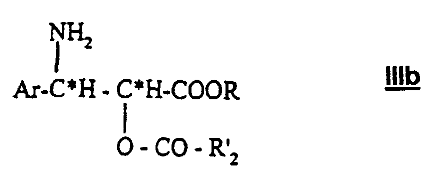 in der Ar, R und R'₂ wie in Anspruch 1 definiert sind,
zu erhalten, das dann gegebenenfalls in das entsprechende Amid der allgemeinen Formel III'b 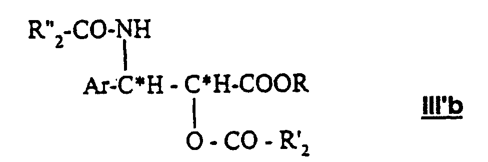 in der Ar, R, R'₂ und R"₂ wie in Anspruch 1 definiert sind, umgewandelt wird.

10. Verfahren gemäß einem der Ansprüche 1 bis 9, **dadurch gekennzeichnet, dass** das cis-β-Aryl-glycidatderivat der allgemeinen Formel I 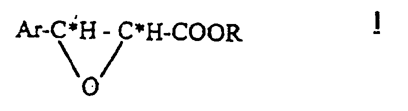 in der
Ar wie in Anspruch 1 definiert ist und
R ein optisch reines Enantiomer eines chiralen Kohlenwasserstoffrests mit großem sterischen Raumbedarf darstellt,
hergestellt wird, indem man den Aldehyd der Formel
Ar-CHO
mit dem Halogenacetat der Formel
X-CH₂-COOR
umsetzt, wobei
Ar, R wie in Anspruch 1 definiert sind und
X ein Halogenatom, insbesondere ein Chlor- oder ein Bromatom, darstellt.

11. Verfahren gemäß einem der Ansprüche 1 bis 10, **dadurch gekennzeichnet, dass** man durch schonende Verseifung die Derivate der Formeln IIa, II'a, IIb, IIIa, III'a, IIIb und III'b, definiert wie in den Ansprüchen 1, 7, 8 und 9, für die R ein Wasserstoffatom darstellt, erhält.

12. Vorstufen von Seitenketten von Taxanen, **dadurch gekennzeichnet, dass** sie unter den Derivaten der folgenden allgemeinen Formeln I, IIa, IIb, II'a, IIIb und III'b ausgewählt sind: 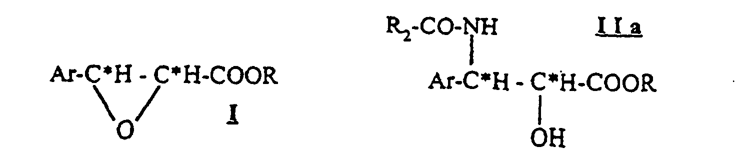 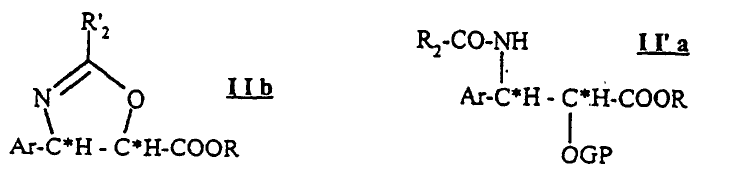 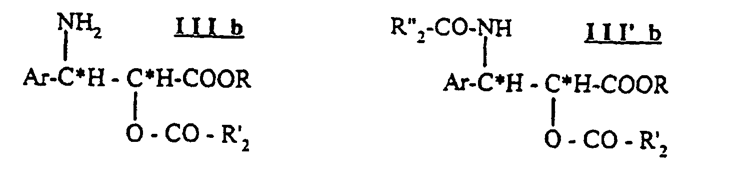 in denen
Ar, R₂, R'₂, R"₂ und GP wie in einem der Ansprüche 1 bis 3 und 5 definiert sind und
R ein optisch reines Enantiomer eines chiralen Kohlenwasserstoffrests mit großem sterischen Raumbedarf darstellt.

13. Verbindungen gemäß Anspruch 12, **dadurch gekennzeichnet, dass** R eines der Enantiomere des Menthylrests, insbesondere (+)-Menthyl, ist.

14. Verbindungen gemäß einem der Ansprüche 12 oder 13, **dadurch gekennzeichnet, dass** das cis-β-Phenyl-glycidatderivat der allgemeinen Formel I die (2R,3R)-Konfiguration hat und die Derivate der allgemeinen Formeln IIa, IIb, IIIb und III'b die (2R,3S)-Konfiguration haben.

15. Vorstufen von Seitenketten von Taxanen, **dadurch gekennzeichnet, dass** sie unter den Derivaten der folgenden allgemeinen Formeln IIIa und III'a ausgewählt sind: 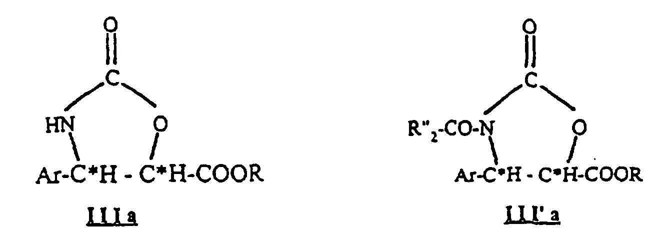 in denen
Ar, R und R"₂ wie in den Ansprüchen 1 und 8 definiert sind oder R ein Wasserstoffatom darstellt.

16. Verbindungen gemäß Anspruch 15, **dadurch gekennzeichnet, dass** sie die (2R,3S)-Konfiguration haben.

17. Verfahren zur Herstellung von Taxanen der allgemeinen Formel IV,
C-B **IV**
in der
B einen Rest der allgemeinen Formel V 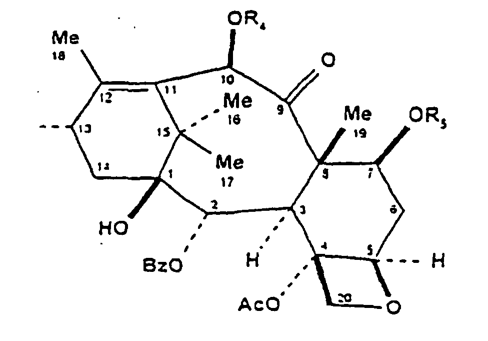 darstellt, in der
Ac den Acetylrest darstellt,
Bz den Benzylrest darstellt,
Me den Methylrest darstellt,
R₄ einen Acetylrest oder eine Schutzgruppe für die Hydroxyfunktion GP1 darstellt und
R₅ eine Schutzgruppe für die Hydroxyfunktion GP2 darstellt und
C eine Seitenkette darstellt, die unter den Resten der folgenden Formeln IIa, II'a, IIb, IIIa, III'a, IIIb und III'b ausgewählt ist: 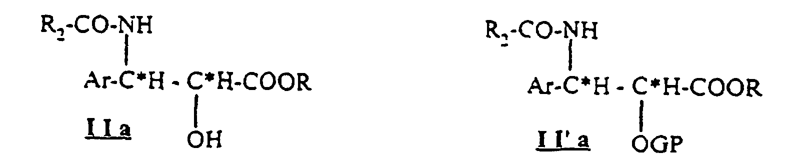 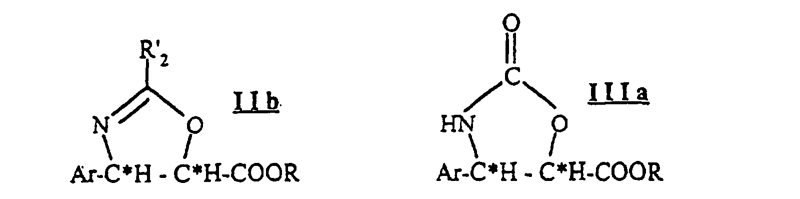 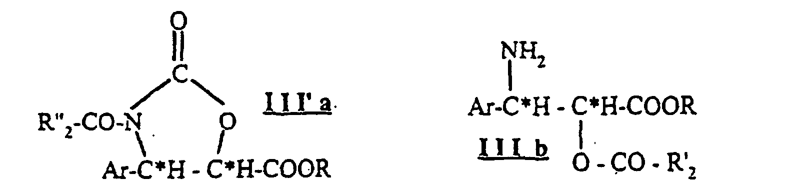 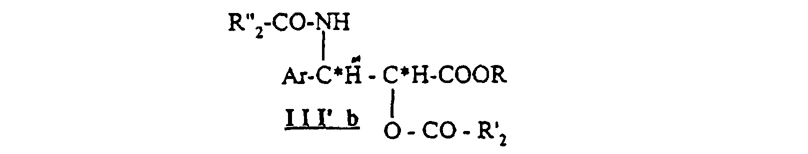
in denen Ar, R₂, R'₂, R"₂ und GP wie in den Ansprüchen 1, 7 und 8 definiert sind, durch Veresterung eines geeigneten Baccatin III-Derivats der allgemeinen Formel V, das eine Hydrowfunktion in Position C 13 trägt, mit einem der Derivate der Formeln IIa, II'a, IIb, IIIa, III'a, IIIb und III'b, für die R ein Wasserstoffatom darstellt, die durch das Verfahren gemäß Anspruch 11 erhalten werden.

18. Verfahren gemäß Anspruch 17, **dadurch gekennzeichnet, dass** die Schutzgruppen GP1 und GP2 unabhängig voneinander übliche Schutzgruppen sind, die bei der Hemisynthese der Taxane verwendet werden, wie die Trialkylsilylgruppen oder TROC, oder sperrige, lineare oder verzweigte Halogenalkoxycarbonylreste mit wenigstens einem Halogenatom, Acylreste, deren Kohlenstoffatom in Position a zur Carbonylfunktion wenigstens ein Sauerstoffatom trägt, ein Trialkylgermanylrest, oder GP1 und GP2 zusammen einen zweiwertigen Rest der Formel
-SiR₇-O-SiR₈-
in der
R₇ und R₈ unabhängig voneinander einen sterisch sperrigen Alkylrest darstellen,
bilden.

19. Verfahren gemäß einem der Ansprüche 17 oder 18, **dadurch gekennzeichnet, dass** die Acylreste, deren Kohlenstoffatom in Position a zur Carbonylfunktion wenigstens ein Sauerstoffatom trägt, unter den
- Alkoxy- oder Aryloxyacetylresten der Formel
R₆-O-CH₂-CO-
in der R₆ einen sterisch sperrigen Alkylrest, einen Cycloalkylrest oder einen Arylrest darstellt,
- oder den Arylidendioxyacetylresten der Formel 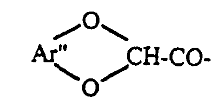 in der Ar" einen Arylidenrest darstellt,
ausgewählt sind.

20. Verfahren gemäß Anspruch 19, **dadurch gekennzeichnet, dass**:
der sterisch sperrige Alkylrest ein linearer oder verzweigter C₁-C₆-Alkylrest ist, der mit einem oder mehreren sperrigen Substituenten substituiert ist, die unter den Halogenen, den linearen oder verzweigten C₁-C₆-Alkylresten, linearen oder verzweigten C₁-C₆-Alkoxyresten oder C₃-C₆-Cycloalkylresten oder Arylresten ausgewählt sind,
der Cycloalkylrest ein C₃-C₆-Cycloalkylrest ist, der gegebenenfalls mit einem oder mehreren sperrigen Substituenten substituiert ist, die unter den Halogenen, den linearen oder verzweigten C₁-C₆-Alkylresten, linearen oder verzweigten C₁-C₆-Alkoxyresten oder Arylresten ausgewählt sind, vorzugsweise ein Cyclohexylrest, der mit einem oder mehreren, linearen oder verzweigten C₁-C₆-Alkylresten substituiert ist, beispielsweise der Menthylrest, dessen Racemat oder dessen Enantiomere und deren Gemische in allen Verhältnissen,
der Arylrest ein Phenyl-, Naphthyl-, Anthryl- oder Phenanthrylrest ist, der gegebenenfalls mit einem oder mehreren sperrigen Substituenten substituiert ist, die unter den Halogenen, den linearen oder verzweigten C₁-C₆-Alkylresten, linearen oder verzweigten C₁-C₆-Alkoxyresten oder Arylresten, insbesondere Phenyl, ausgewählt sind, vorzugsweise ein Phenylrest, der gegebenenfalls mit einem oder zwei der obigen sperrigen Substituenten in ortho- und ortho'-Stellung zur Etherbindung substituiert ist, und
der Arylidenrest ein Phenylen-, Naphthylen-, Anthrylen- oder Phenanthrylenrest ist, der gegebenenfalls mit einem oder mehreren sperrigen Substituenten substituiert ist, die unter den Halogenen, den linearen oder verzweigten C₁-C₆-Alkylresten, linearen oder verzweigten C₁-C₆-Alkoxyresten oder Arylresten, insbesondere Phenyl, ausgewählt sind.

21. Verfahren gemäß einem der Ansprüche 17 oder 18, **dadurch gekennzeichnet, dass** R₄ einen Acetylrest darstellt und GP2 einen Trialkylsilyl-, 2,2,2-Trichlorethoxycarbonyl-, 2,2,2-Tribromethoxycarbonyl-, 2,2,2,1-Tetrachlorethoxycarbonyl-, 2,2,2-Trichlor-tert.-butoxycarbonyl-, Trichlormethoxycarbonyl-, Phenoxyacetyl- oder Trialkyl-germanylrest darstellt.

22. Verfahren gemäß einem der Ansprüche 17 oder 18, **dadurch gekennzeichnet, dass** R₄ eine Gruppe GP1 darstellt und GP1 und GP2 einen 2,2,2-Trichlorethoxycarbonyl- oder Phenoxyacetylrest darstellen oder zusammen einen zweiwertigen Rest der Formel
-SiR₇-O-SiR₈-
in der R₇ und R₈ jeweils einen Isopropylrest darstellen, bilden.

23. Verfahren gemäß einem der Ansprüche 17 bis 21, **dadurch gekennzeichnet, dass** C einen Rest der Formel IIa darstellt, wobei Ar und R₂ einen Phenylrest darstellen, und R₄ einen Acetylrest darstellt.

24. Verfahren gemäß einem der Ansprüche 17 bis 23, **dadurch gekennzeichnet, dass** man dann die Schutzgruppen für die Hydroxyfunktionen der Derivate der allgemeinen Formel IV entfernt und gegebenenfalls, gleichzeitig oder getrennt, den Oxazolinring der Reste der Formel IIb oder IIIa öffnet, um ein Taxanderivat der allgemeinen Formel VI 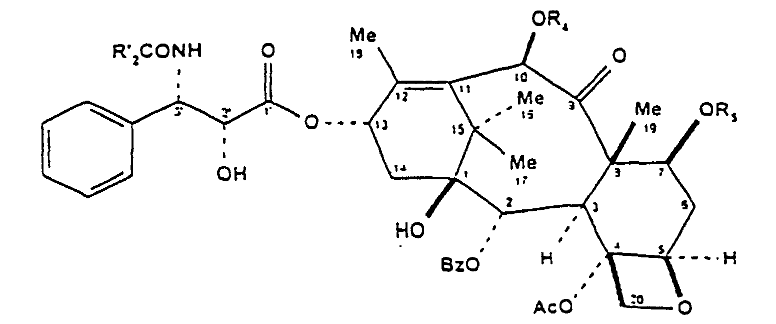 in der
Ac, Bz, Me und R'₂ wie in einem der vorigen Ansprüche definiert sind,
R₄ ein Wasserstoffatom oder einen Acetylrest darstellt und
R₅ ein Wasserstoffatom darstellt,
zu erhalten.

25. Taxanderivate der allgemeinen Formel IV
C-B **IV**
in der C und B wie in einem der Ansprüche 17 bis 23 definiert sind, mit Ausnahme der Derivate, für die C einen Rest der Formel lla, II'a, llb, IIIb oder III'b darstellt, und GP1 und/oder GP2 unabhängig voneinander übliche Gruppen sind, die bei der Hemisynthese der Taxane verwendet werden, wie die Trialkylsilylgruppen oder TrOC.

26. Baccatin III-Derivate, die für die Hemisynthese von Taxanen nützlich sind, **dadurch gekennzeichnet, dass** sie unter den Derivaten der allgemeinen Formel V, 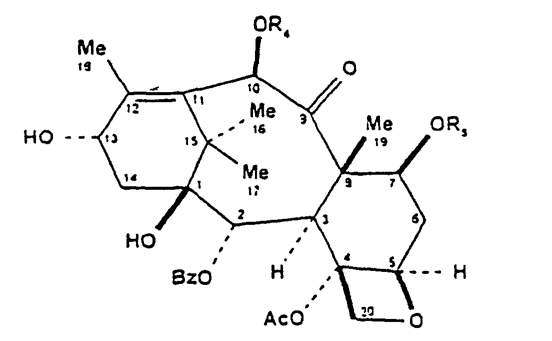 ausgewählt sind, in der
Ac den Acetylrest darstellt,
Bz den Benzoylrest darstellt,
Me den Methylrest darstellt,
R₄ einen Acetylrest oder eine Schutzgruppe für die Hydroxyfunktion GP1 darstellt,
R₅ eine Schutzgruppe für die Hydroxyfunktion GP2 darstellt und
GP1 und GP2 unabhängig voneinander sperrige Halogenalkoxycarbonylreste mit Ausnahme von TrOC, Acylreste, deren Kohlenstoffatom in Position a zur Carbonylfunktion wenigstens ein Sauerstoffatom trägt, Trialkylgermanylreste sind oder GP1 und GP2 zusammen einen zweiwertigen Rest der Formel
-SiR₇-O-SiR₈-
in der
R₇ und R₈ unabhängig voneinander einen sterisch sperrigen Alkylrest darstellen, bilden.
